# EUROPEAN PATENT APPLICATION

(11) **EP 3 444 331 A1**
(43) Date of publication of application: **20.02.2019**
(21) Application number: 17186131.3
(22) Date of filing: 14.08.2017
(51) Int. Cl.: C12N 5/077

(54) **FACILITATED GENERATION OF CARDIOMYOCYTES BY FORWARD PROGRAMMING OF HUMAN PLURIPOTENT STEM CELLS**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a method for the production of cells having a cardiomyocyte phenotype, comprising: (a) overexpression of EOMES in embryonic stem cells or induced pluripotent stem cells for about 1 to 6 days, preferably 2 to 4 days and most preferably about 3 days, and (b) 24h to 96h, preferably 48 to 72h after the overexpression of (a), inhibition of the WNT-pathway for at least 24h thereby differentiating said embryonic stem cells or induced pluripotent stem cells into cells having a cardiomyocyte phenotype.

SEQUENCE LISTING

<110> Max-Planck-Gesellschaft zur Förderung der Wissenschaften e.V.

<120> Facilitated generation of cardiomyocytes by forward programming of
human pluripotent stem cells

<130> AA1877 EP

<160> 72

<170> BiSSAP 1.3.6

<210> 1
<211> 23
<212> DNA
<213> Artificial Sequence

<220>
<223> ACTN2 Fwd primer

<400> 1
gccagagaga aggatgcaat cac                                              23

<210> 2
<211> 22
<212> DNA
<213> Artificial Sequence

<220>
<223> ACTN2 Rev primer

<400> 2
aagcatggga acctggaatc aa                                               22

<210> 3
<211> 20
<212> DNA
<213> Artificial Sequence

<220>
<223> CACNA1C Fwd primer

<400> 3
ccaggctcca cgacttcaca                                                  20

<210> 4
<211> 20
<212> DNA
<213> Artificial Sequence

<220>
<223> CACNA1C Rev primer

<400> 4
ggcctttctc gagggtgaga                                                  20

<210> 5
<211> 20
<212> DNA
<213> Artificial Sequence

<220>
<223> CDX1 Fwd primer

<400> 5
tgaggaggga ggaacgtggt                                                    20

<210> 6
<211> 21
<212> DNA
<213> Artificial Sequence

<220>
<223> CDX1 Rev primer

<400> 6
gggctcagtg cccttatgat g                                                  21

<210> 7
<211> 21
<212> DNA
<213> Artificial Sequence

<220>
<223> CTNT Fwd primer

<400> 7
ggcagctcct gtttggaaat g                                                  21

<210> 8
<211> 25
<212> DNA
<213> Artificial Sequence

<220>
<223> CTNT Rev primer

<400> 8
ttattactgg tgtggagtgg gtgtg                                              25

<210> 9
<211> 20
<212> DNA
<213> Artificial Sequence

<220>
<223> DHRS9 Fwd primer

<400> 9
tccggtggta gagtgcatgg                                                    20

<210> 10
<211> 19
<212> DNA
<213> Artificial Sequence

<220>
<223> DHRS9 Rev primer

<400> 10
gcaaagctgc tggcatgtg                                                     19

<210> 11
<211> 18
<212> DNA
<213> Artificial Sequence

<220>
<223> EOMES CDS Fwd primer

<400> 11
cggcctctgt ggctcaaa                                                      18

<210> 12
<211> 18
<212> DNA
<213> Artificial Sequence

<220>
<223> EOMES CDS Rev primer

<400> 12
aaggaaacat gcgcctgc                                                      18

<210> 13
<211> 22
<212> DNA
<213> Artificial Sequence

<220>
<223> EOMES UTR Fwd primer

<400> 13
cttgctaggc ctctgctgtg tg                                                 22

<210> 14
<211> 25
<212> DNA
<213> Artificial Sequence

<220>

<223> EOMES UTR Rev primer

<400> 14
ttggtgactc cttagcttgc tctct                                          25


<210> 15
<211> 20
<212> DNA
<213> Artificial Sequence


<220>
<223> IRX4 Fwd primer

<400> 15
cggagcagaa gaggccagat                                                20


<210> 16
<211> 20
<212> DNA
<213> Artificial Sequence


<220>
<223> IRX4 Rev primer

<400> 16
cagaacggaa ccgccttctc                                                20


<210> 17
<211> 22
<212> DNA
<213> Artificial Sequence


<220>
<223> KCNA5 Fwd primer

<400> 17
ccagcagagg gataacccaa ac                                             22


<210> 18
<211> 22
<212> DNA
<213> Artificial Sequence


<220>
<223> KCNA5 Rev primer

<400> 18
ttggaacaca tggatggagg ag                                             22


<210> 19
<211> 20
<212> DNA
<213> Artificial Sequence

<220>
<223> KCNH2 Fwd primer

<400> 19
cggtgcatgt gtggtcttga                                                    20


<210> 20
<211> 20
<212> DNA
<213> Artificial Sequence


<220>
<223> KCNH2 Rev primer

<400> 20
tgacatctgc ctgcacctga                                                    20


<210> 21
<211> 19
<212> DNA
<213> Artificial Sequence


<220>
<223> KCNQ1 Fwd primer

<400> 21
gcagccagcc aaacacaca                                                     19


<210> 22
<211> 21
<212> DNA
<213> Artificial Sequence


<220>
<223> KCNQ1 Rev primer

<400> 22
gcgatgtaat gcccagaagg a                                                  21


<210> 23
<211> 23
<212> DNA
<213> Artificial Sequence


<220>
<223> MSX1 Fwd primer

<400> 23
aaccctcaca ctgctccagt ttc                                                23


<210> 24
<211> 21
<212> DNA

<213> Artificial Sequence

<220>
<223> MSX1 Rev primer

<400> 24
tttggcaggg atcagacttc g                                                21

<210> 25
<211> 20
<212> DNA
<213> Artificial Sequence

<220>
<223> MYH6 Fwd primer

<400> 25
acctggtgga caagctgcaa                                                  20

<210> 26
<211> 20
<212> DNA
<213> Artificial Sequence

<220>
<223> MYH6 Rev primer

<400> 26
caccttgcgg aacttggaca                                                  20

<210> 27
<211> 20
<212> DNA
<213> Artificial Sequence

<220>
<223> MYH7 Fwd primer

<400> 27
ttgatctgct cagccctgga                                                  20

<210> 28
<211> 21
<212> DNA
<213> Artificial Sequence

<220>
<223> MYH7 Rev primer

<400> 28
gcttcctccc aaggagctgt t                                                21

<210> 29

<211> 20
<212> DNA
<213> Artificial Sequence

<220>
<223> MYL2 / MLC2v Fwd primer

<400> 29
tggtccctgc cctcatctct                                                    20

<210> 30
<211> 20
<212> DNA
<213> Artificial Sequence

<220>
<223> MYL2 / MLC2v Rev primer

<400> 30
ggcagccaca tggctaacag                                                    20

<210> 31
<211> 20
<212> DNA
<213> Artificial Sequence

<220>
<223> MYL3 Fwd primer

<400> 31
aagttgatgg ctgggcaaga                                                    20

<210> 32
<211> 20
<212> DNA
<213> Artificial Sequence

<220>
<223> MYL3 Rev primer

<400> 32
gcacgaggtt tagctggaca                                                    20

<210> 33
<211> 20
<212> DNA
<213> Artificial Sequence

<220>
<223> MYL4 Fwd primer

<400> 33
ctgggcaaga ggatgccaat                                                    20

<210> 34
<211> 22
<212> DNA
<213> Artificial Sequence

<220>
<223> MYL4 Rev primer

<400> 34
gcacctggaa gactctgctt ca                                                22

<210> 35
<211> 20
<212> DNA
<213> Artificial Sequence

<220>
<223> NKX2-5 Fwd primer

<400> 35
accgatccca cctcaacagc                                                   20

<210> 36
<211> 20
<212> DNA
<213> Artificial Sequence

<220>
<223> NKX2-5 Rev primer

<400> 36
ctccgcagga gtgaatgcaa                                                   20

<210> 37
<211> 22
<212> DNA
<213> Artificial Sequence

<220>
<223> NPPA / ANP Fwd primer

<400> 37
gctgcagctt cctgtcaaca ct                                                22

<210> 38
<211> 21
<212> DNA
<213> Artificial Sequence

<220>
<223> NPPA / ANP Rev primer

<400> 38

aggcgaggaa gtcaccatca a                                                21

<210> 39
<211> 23
<212> DNA
<213> Artificial Sequence

<220>
<223> RPL37A Fwd primer

<400> 39
gtggttcctg catgaagaca gtg                                              23

<210> 40
<211> 21
<212> DNA
<213> Artificial Sequence

<220>
<223> RPL37A Rev primer

<400> 40
ttctgatggc ggactttacc g                                                21

<210> 41
<211> 20
<212> DNA
<213> Artificial Sequence

<220>
<223> RYR2 Fwd primer

<400> 41
ggagccagtg tcatccacca                                                  20

<210> 42
<211> 22
<212> DNA
<213> Artificial Sequence

<220>
<223> RYR2 Rev primer

<400> 42
caggtggctg aaagaatgag ca                                               22

<210> 43
<211> 21
<212> DNA
<213> Artificial Sequence

<220>
<223> SCN5A Fwd primer

<400> 43
actgcacaat gaccagcagg a                                                21

<210> 44
<211> 27
<212> DNA
<213> Artificial Sequence

<220>
<223> SCN5A Rev primer

<400> 44
gtgagaagtg ctcgattagt tcagaca                                          27

<210> 45
<211> 23
<212> DNA
<213> Artificial Sequence

<220>
<223> SHOX2 Fwd primer

<400> 45
tctgtatccc aattcgctag caa                                              23

<210> 46
<211> 23
<212> DNA
<213> Artificial Sequence

<220>
<223> SHOX2 Rev primer

<400> 46
caaccgcata ccaaagttca gtt                                              23

<210> 47
<211> 20
<212> DNA
<213> Artificial Sequence

<220>
<223> SP5 Fwd primer

<400> 47
ttccaacttc cgctgccttc                                                  20

<210> 48
<211> 20
<212> DNA
<213> Artificial Sequence

<220>
<223> SP5 Rev primer

<400> 48
cgtacggcag agctcccaat                                                    20


<210> 49
<211> 22
<212> DNA
<213> Artificial Sequence


<220>
<223> T Fwd primer

<400> 49
ccttgctcac acctgcagta gc                                                 22


<210> 50
<211> 20
<212> DNA
<213> Artificial Sequence


<220>
<223> T Rev primer

<400> 50
ggccaactgc atcatctcca                                                    20


<210> 51
<211> 20
<212> DNA
<213> Artificial Sequence


<220>
<223> WNT3 1/1' Fwd primer

<400> 51
ggctcacagc ccctgctctg                                                    20


<210> 52
<211> 20
<212> DNA
<213> Artificial Sequence


<220>
<223> WNT3 1/1' Rev primer

<400> 52
gagatgtgta ctgctggccc                                                    20


<210> 53
<211> 20
<212> DNA
<213> Artificial Sequence

<220>
<223> WNT3 2/2′ Fwd primer

<400> 53
gagggttcac aaagcggcca                                                    20


<210> 54
<211> 20
<212> DNA
<213> Artificial Sequence


<220>
<223> WNT3 2/2′ Rev primer

<400> 54
ggggtaggtg gagaggcaga                                                    20


<210> 55
<211> 20
<212> DNA
<213> Artificial Sequence


<220>
<223> WNT3A 1/1′ Fwd primer

<400> 55
gctacagacc ctttgctctg                                                    20


<210> 56
<211> 20
<212> DNA
<213> Artificial Sequence


<220>
<223> WNT3A 1/1′ Rev primer

<400> 56
gctcatctgt gtcgctgtcc                                                    20


<210> 57
<211> 20
<212> DNA
<213> Artificial Sequence


<220>
<223> WNT3A 2/2′ Fwd primer

<400> 57
gatgggctgc gagcccaggg                                                    20


<210> 58
<211> 20

<212> DNA
<213> Artificial Sequence

<220>
<223> WNT3A 2/2' Rev primer

<400> 58
gtgtgccagc atcccgggcc                                                    20

<210> 59
<211> 18
<212> DNA
<213> Artificial Sequence

<220>
<223> WNT3 span ex2 Fwd primer

<400> 59
cagggtcctc gctggcta                                                      18

<210> 60
<211> 20
<212> DNA
<213> Artificial Sequence

<220>
<223> WNT3 span ex2 Rev primer

<400> 60
gcctcgttgt tgtgcttgtt                                                    20

<210> 61
<211> 20
<212> DNA
<213> Artificial Sequence

<220>
<223> WNT3A span ex2 Fwd primer

<400> 61
gccccactcg gatacttctt                                                    20

<210> 62
<211> 20
<212> DNA
<213> Artificial Sequence

<220>
<223> WNT3A span ex2 Rev primer

<400> 62
accatcccac caaactcgat                                                    20

<210> 63
<211> 20
<212> DNA
<213> Artificial Sequence

<220>
<223> WNT3 in ex2 Fwd primer

<400> 63
gggccagcag tacacatctc                                                    20

<210> 64
<211> 20
<212> DNA
<213> Artificial Sequence

<220>
<223> WNT3 in ex2 Rev primer

<400> 64
gcctcgttgt tgtgcttgtt                                                    20

<210> 65
<211> 18
<212> DNA
<213> Artificial Sequence

<220>
<223> WNT3A in ex2 Fwd primer

<400> 65
cagtattcct ccctgggc                                                      18

<210> 66
<211> 20
<212> DNA
<213> Artificial Sequence

<220>
<223> WNT3A in ex2 Rev primer

<400> 66
accatcccac caaactcgat                                                    20

<210> 67
<211> 20
<212> DNA
<213> Artificial Sequence

<220>
<223> WNT3 promoter Fwd primer

<400> 67
ggacggagcc gagtgtcatt                                                    20

<210> 68
<211> 20
<212> DNA
<213> Artificial Sequence

<220>
<223> WNT3 promoter Rev primer

<400> 68
atgcaaaggc agcaggaggt                                                    20

<210> 69
<211> 21
<212> DNA
<213> Artificial Sequence

<220>
<223> WNT3A promoter Fwd primer

<400> 69
cacaccacgg atgagtctgg a                                                  21

<210> 70
<211> 20
<212> DNA
<213> Artificial Sequence

<220>
<223> WNT3A promoter Rev primer

<400> 70
accgcgttgg aattgaggaa                                                    20

<210> 71
<211> 21
<212> DNA
<213> Artificial Sequence

<220>
<223> OTX2 Fwd primer

<400> 71
tctgcctttg tcttgggatg c                                                  21

<210> 72
<211> 21
<212> DNA
<213> Artificial Sequence

<220>
<223> OTX2 Rev primer

<400> 72
gcccattctt ctgccaattc a 21

## Description

The present invention relates to a method for the production of cells having a cardiomyocyte phenotype, comprising: (a) overexpression of EOMES in embryonic stem cells or induced pluripotent stem cells for about 1 to 6 days, preferably 2 to 4 days and most preferably about 3 days, and (b) 24h to 96h, preferably 48 to 72h after the overexpression of (a), inhibition of the WNT-pathway for at least 24h thereby differentiating said embryonic stem cells or induced pluripotent stem cells into cells having a cardiomyocyte phenotype.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Cardiomyocytes are the muscle cells that make up the cardiac muscle. Each myocardial cell contains myofibrils, which are specialized organelles consisting of long chains of sarcomeres, the fundamental contractile units of muscle cells. Cardiomyocytes show striations similar to those on skeletal muscle cells. Unlike multinucleated skeletal cells, the majority of cardiomyocytes contain only one nucleus, although they may have as many as four. Cardiomyocytes have a high mitochondrial density, which allows them to produce adenosine triphosphate (ATP) quickly, making them highly resistant to fatigue.

Embryonic stem cells (ESCs) were first successfully differentiated into cell having a cardiomyocyte phenotype more than a decade ago. Stem cell-derived cardiomyocytes are a novel source of cells for patient-specific cardiotoxicity drug testing, drug discovery, disease modeling, and regenerative medicine. All these applications require a routine supply of relatively large quantities of *in vitro-*generated cardiomyocytes (Kempf et al., Nature Protocols, 10:1345-1361 (2015)). For instance, methods for obtaining stem cell-derived cardiomyocytes permit the study of human heart development in ways not otherwise possible. Stem cell-derived cardiomyocytes can also serve as a cardiac model that can be used for diverse basic research studies ranging from cellular electrophysiology to protein biochemistry. Furthermore, the ability to generate stem cell-derived cardiomyocytes from patients with inherited cardiac diseases provides unprecedented opportunities for studying disease in human cardiomyocytes. Access to abundant populations of human stem cell-derived cardiomyocytes is of particular interest to the pharmaceutical industry as a tool to develop new cardioactive compounds, and perhaps more importantly, to screen compounds for potential cardiotoxicity. Finally, clinical applications using stem cell-derived cardiomyocytes might provide a powerful approach to repair the injured heart (Mummery et al., Circ Res. 2012; 111(3): 344-358).

Since the first successful differentiation of ESCs into cells having a cardiomyocyte phenotype the differentiation process was further enhanced over recent years by the identification of further methods to induce cardiomyocytes. However the currently available differentiation protocols still lag behind the great need of stem cell-derived cardiomyocytes for the various applications described herein above. In many cases the stem cell-derived cardiomyocytes are obtained at low efficiency and require additional enrichment procedures, or the underlying procedures lack a high degree of reproducibility, or they require expensive media additives and growth factors.

There is consequently an ongoing need for means and methods for the efficient, robust, and cost-effective directed differentiation of stem cells into cells having a cardiomyocyte phenotype. This need is addressed by the present invention.

Thus, the present invention relates in a first aspect to a method for the production of cells having a cardiomyocyte phenotype, comprising: (a) overexpression of EOMES in embryonic stem cells or induced pluripotent stem cells for about 1 to 6 days, preferably 2 to 4 days and most preferably about 3 days, and (b) 24h to 96h, preferably 48 to 72h after the overexpression of (a), inhibition of the WNT-pathway for at least 24h thereby differentiating said embryonic stem cells or induced pluripotent stem cells into cells having a cardiomyocyte phenotype.

The term "cardiomyocyte", as used herein, is defined in accordance with the pertinent art and relates to a cardiac muscle cell. Cardiomyocytes are the cells responsible for generating contractile force in the intact heart.

The term "cells having a cardiomyocyte phenotype" takes into account that the cells produced by the method of the invention closely resemble but are not 100% identical to naturally occurring cardiomyocytes. As will be further detailed herein below, while the cells having a cardiomyocyte phenotype obtained by the above method share morphological characteristics and the expression of cardiomyocyte-specific genes with naturally occurring cardiomyocytes. Certain genes being highly expressed in naturally occurring cardiomyocytes were found at an significantly reduced expression level in the cells having a cardiomyocyte phenotype obtained by the above method.

As used herein embryonic stem cells (ESCs) designate pluripotent stem cells derived from the inner cell mass of a blastocyst, an early-stage preimplantation embryo. Human embryos reach the blastocyst stage 4-5 days post fertilization, at which time they consist of 50-150 cells. Isolating the embryoblast or inner cell mass (ICM) may result in the destruction of the blastocyst. However, it is also possible to extract embryonic stem cells without destroying the actual embryo; see, for example, Klimanskaya et al., (2006), Nature. 444 (7118): 481-5 and Chung Y et al., (2008), Cell Stem Cell 2: 113-117. Accordingly, the embryonic stem cells and in particular any human embryonic stem cells to be used herein are preferably those that were obtained without destruction of the embryo.

Induced pluripotent stem cells are a type of pluripotent stem cell that can be generated directly from adult cells. The iPSC technology was established in 2006. The introduction of four specific genes encoding transcription factors was used to convert adult cells into pluripotent stem cells; Takahashi and Yamanaka, (2006), Cell, 126 (4): 663-76. Pluripotent stem cells hold great promise in the field of regenerative medicine. They can propagate indefinitely and can under appropriate differentiation conditions give rise to every other cell type in the body including cardiomyocytes. Since iPSCs can be derived directly from adult tissues, they not only bypass the need for embryos, but can be made in a patient-matched manner. This means that each individual could have their own pluripotent stem cell line, so that potential graft-versus-host reactions can be avoided.

ESCs and iPSCs are preferably mammalian embryonic stem cells and mammalian induced pluripotent stem cells and are most preferably human embryonic stem cells (hESCs) and human induced pluripotent stem cells (hiPSCs). Mammals in accordance with the present invention include all mammals, in particular human, domestic and farm animals, experimental animals as well as wild animals. Preferred examples are experimental animals, such as mouse, rat, dog, pig and monkeys. Human is the most preferred mammal.

The method of the invention is carried out under suitable cell culture conditions. In accordance with the present invention, any basic medium suitable as a culture medium for embryonic stem cells or induced pluripotent stem cells may be employed. It is well established in the art that induced iPSCs and ESCs can be cultured on coated plates with a supporting layer of feeder cells, such as mouse embryonic fibroblasts (MEF) or human foreskin fibroblasts (HFF), or on an extracellular matrix, such as a basement membrane gel, or on recombinant vitronectin-coated surfaces. The medium may be supplemented with serum, including non-human or human serum. Preferred amounts of serum to be employed are between 1% and 30%, more preferably between 2 and 20%, such as e.g. between 5 and 15% and most preferably the amount is about 10%. Serum such as for example FCS may be obtained from e.g. GIBCO, Sigma or PAA. However, meanwhile also feeder-independent and/or serum-free media for iPSCs and ESCs are available; see, for example, Vallier, Methods Mol Biol, 2011; 690:57-66 and Martin Gonzalez et al., Stem Cell Reports, 2016; 7(2):177-91. In addition, stem cell growth media can be supplemented with growth factors. Preferably, the cell culture conditions comprise conditions of about 2 to 10% CO₂ preferably about 5% CO₂ and a temperature of about 32 to 38°C, preferably about 37°C. Preferably, the cell culture is carried out under sterile conditions. The media may be changed, e.g., daily and monitored for overgrowth. Exemplary culture conditions for HuES6 human embryonic stem cells are provided in the examples herein below.

The method of the invention can in principle be applied to ESCs or iPSCs grown in solution as well as adherently, e.g. on the ground of a cell culture flask. The ESCs or iPSCs are preferably grown adherently. Moreover, the adherent cell density of the ESCs or iPSCs in the method of the invention and at the time-point of differentiation initiation is preferably 100,000 to 600,000 cells/cm², more preferably 200,000 to 500,000 cells/cm² and most preferably 300,000 to 400,000 cells/cm².

Eomesodermin (EOMES; also known as T-box brain protein 2 (Tbr2)) is a protein that in humans is encoded by the EOMES gene. EOMES is a member of a conserved protein family that shares a common DNA-binding domain, the T-box. T-box genes encode transcription factors, which control gene expression, involved in the regulation of developmental processes.

As used herein the term "overexpression" requires that a cell is manipulated so that a gene product is expressed in the cell - in the present case the mRNA encoding EOMES - at an expression level being superior as compared to expression level in the corresponding non-manipulated cell. The term "expression level" refers to the number of mRNA copies within a cell encoding a particular gene product. Expression levels are preferably not determined in an absolute manner but in a relative manner, e.g., in comparison to one or more so-called house keeping genes. Non-limiting examples of house keeping genes are CASC3, EIF2B1, IPO8, MRPL19, PGK1, POP4, PPIA, RPL37A, GAPDH and ACTB. Means for determining gene expression levels are established in the art and include, for example, northern blot, serial analysis of gene expression (SAGE), microarray analysis, RNA-sequencing, and quantitative RT-PCR (qPCR).

qPCR is the preferred technique. qPCR utilizes specific complementary oligonucleotide primers that amplify the target of interest in combination with a fluorescent readout, based either on DNA intercalating dyes or hydrolysis-based probes (with hydrolysis probes providing additional specificity for highly stringent assays). qPCR can be used to determine absolute and relative quantification expression levels. Digital PCR (dPCR) is a qPCR that allows absolute quantification. Digital PCR measurements are performed by dividing the sample and qPCR assay mixture into a very large number of separate small volume reactions, such that there is either zero or one target molecule present in any individual reaction. This is the fundamental concept for making "digital" measurements. In absolute quantification using digital PCR, no known standards are needed. The target of interest can be directly quantified with precision determined by the number of digital PCR replicates. Quantitative qPCR can also be performed by using the standard curve method, wherein the unknown expression level is determined based on a known quantity as the standard of comparison. First a standard curve is created; the determined unknown levels are compared to the standard curve and an absolute expression level is extrapolated as a value. In a relative qPCR, the gene expression level in a given sample is determined relative to another reference sample, preferably one or more house keeping genes.

The overexpression is preferably an inducible overexpression, which allows switching the overexpression in the cell on and off, as needed. Systems for inducible expression are widely used in the state of the art and include but are not limited to the Tet-on system, Tet-off system, lac operator/expressor system and the commercial systems Q-mate, RheoSwitch, Cumate or TREX. As known in the art, inducible expression systems allow to control the expression of a gene over time, the order of the genes expressed (provided that different expression systems, e.g. a Tet-on system and Tet-off system are used in parallel or subsequently for the expression of different genes) and also the amount of expressed mRNA.

With respect to the gene expression level of the mRNA encoding EOMES it is preferred that the overexpression of EOMES results in a relative expression level which is above 0.1% and below 10% of the expression level of the house keeping gene RPL37A (Ribosomal Protein L37a) as determined by qPCR. The house keeping gene RPL37A was found to be an optimal single reference gene for determining gene expression levels; see, for example, Pfister et al., BMC Res Notes. 2011; 4:275.

The proteins in the WNT pathway (or WNT signalling pathway) act as intercellular signals. The proteins in the WNT pathway are active in numerous contexts, initially in early development and later during the growth and maintenance of various tissues. In comparison to other growth factors, Wnt signals have several unique properties, including a short range of action. Thereby, via the WNT pathway signalling is predominantly mediated locally, between neighboring cells. In addition, the signals of the WNT pathway give shape to tissues as cells are proliferating. This is a consequence of the ability of Wnt signaling to confer polarity and asymmetry to cells. The proteins in the WNT pathway are highly conserved in evolution and are active in every branch of the animal kingdom. WNT pathway signaling is often implicated in stem cell control, as a proliferative and self-renewal signal. Mutations in Wnt genes or Wnt pathway components may lead to specific developmental defects, while various human diseases, including cancer, are caused by abnormal signalling in the WNT pathway.

The proteins in the WNT pathway *inter alia* comprise the WNT proteins as such, Frizzled (or SFRP), Dishevelled (Dvl), TCF, LRP, APC, β-catenin and Axin; see, for example, MacDonald, Dev Cell., 2009; 17(1):9-26. The human WNT proteins comprise WNT1, WNT2, WNT2B, WNT3, WNT3A, WNT4, WNT5A, WNT5B, WNT6, WNT7A, WNT7B, WNT8A, WNT8B, WNT9A, WNT9B, WNT10A, WNT10B, WNT11, and WNT16. The WNT pathway has been extensively studied and further information on this pathway is available, for example, via "the WNT homepage" of the Stanford University (http://web.stanford.edulgrouplnusselab/cgi-bin/wnt/).

The Wnt signaling pathway is generally classified into the canonical Wnt pathway, the noncanonical planar cell polarity pathway, and the noncanonical Wnt/calcium pathway. These pathways belong to one of two categories: canonical or noncanonical. Whereas the canonical Wnt pathway (or Wnt/β-catenin pathway) causes an accumulation of β-catenin in the cytoplasm and its eventual translocation into the nucleus to act as a transcriptional coactivator of transcription factors that belong to the TCF/LEF family, the noncanonical planar cell polarity (PCP) pathway and the noncanonical Wnt/calcium pathway do not involve β-catenin. In accordance with the present invention the canonical WNT-pathway has to be inhibited. The inhibition of the canonical WNT-pathway may also result in the inhibition of the noncanonical WNT-pathways.

The means to be used for the inhibition of the WNT pathway are not particularly limited. Various ways to specifically inhibit Wnt signalling in cells are known in the art; see again "the WNT homepage" of the Stanford University. Possible mechanisms and/or targets for the inhibition of the WNT pathway are, for example, Tankyrase inhibition, PORCN inhibition, TNIK inhibition, β-catenin/TCF/LEF transcription factors or TF complex inhibition, Axin destruction complex stabilization, and inhibition of β-catenin recruitment.

In order to further illustrate the various ways for the inhibition of the WNT pathway the following non-limiting examples are provided: (i) RNAi targeting various component of the pathway, such as LRP or Dishevelled can be used to inhibit the WNT pathway. This has been shown to work very well for Drosophila cells as well as mammalian cells. (ii) A variety of small molecules are available that inhibit Wnt signaling. In the examples the small molecule WNT inhibitor C-59 is used. Further examples of such small molecules will be provided herein below. (iii) The (secreted) Wnt signal can be blocked by an excess of the ligand binding domain of its receptor, Frizzled. This domain is best made as its natural fusion in the FRP/Frz form. Alternatively, it can be expressed on the surface of target cells using a GPI anchor, which works well. (iv) Another way of inhibiting Wnt is to add excess of Dickkopf (Dkk) protein. Dkk binds to the LRP co-receptor for Wnt. (v) In order to block signaling inside cells, several workers have used dominant negative Dishevelled. (vi) Overexpressing intact Axin, a negative regulator of the Wnt pathway, works also very well. (vii) Likewise, the overexpression of full length GSK can block Wnt signaling effectively. (viii) Dominant negative forms of TCF can be used to block Wnt signaling. (ix) Finally, an antibody to frizzled (e.g. the antibody OMP-18R5) can be used. Such an antibody may interact with multiple Frizzled receptors.

Some of the described means for the inhibition of the WNT pathway may encompass the overexpression of certain factors (e.g. the overexpression the gene encoding ligand binding domain of Frizzled, Dkk or Axin). These factors may be overexpressed by the same means as described herein for EOMES, provided that the temporal order as required by the method according to the first aspect of the invention is met. For instance, also the overexpression of these factors is preferably an inducible overexpression and the induction of the EOMES overexpression and the overexpression of the factor may be induced in accordance with the temporal order as required by the method according to the first aspect of the invention. Certain means for the inhibition of the WNT pathway will be further detailed herein below.

With respect to the time frames of 1 to 6 days, preferably 2 to 4 days and most preferably about 3 days, and 24h to 96h, preferably 48 to 72h it is noted that 1 day corresponds to 24h. The inhibition of the WNT-pathway of at least 24h is preferably carried out for about 48h. As used herein the term "about" means preferably ±20% and preferably ±10%.

As can be taken from the examples herein below it was surprisingly found that in order to efficiently obtain cells having a cardiomyocyte phenotype from embryonic stem cells or induced pluripotent stem cells it is sufficient to overexpress EOMES in combination with inhibiting the WNT-pathway. In more detail, transcriptional master regulators are thought to induce specific cell lineages in gastrulation by orchestrating entire gene programmes^{1, 2}. The T-box transcription factor EOMES is crucially required for the development of the heart - yet it is equally important for endoderm specification suggesting that it may act in a context-dependent manner³⁻⁷. Using loss and gain-of-function approaches in human embryonic stem cells, the examples herein below revealed an unrecognised interplay between EOMES and the WNT signaling pathway in controlling cardiac induction. It was surprisingly found that the overexpression of *EOMES* alone can fully replace a cocktail of signaling molecules otherwise essential for the specification of cardiogenic mesoderm⁸. It was furthermore unexpectedly found that highly efficient cardiomyocyte programming by EOMES mechanistically involves autocrine activation of canonical WNT signaling via the WNT3 ligand, which necessitates an inhibition of the WNT pathway at a stage subsequent to the overexpression of *EOMES -* reinforcing the idea of an interconnection between EOMES and the WNT pathway. In this respect it is emphasized that the method of the invention for obtaining cells having a cardiomyocyte phenotype only works provided that EOMES is first overexpresseed and then in a second step the WNT-pathway is inhibited as is expressed in the method according to the first aspect of the invention. Hence, the sheer combination as well as the timing of performing steps (a) and (b) as recited in the method according to the first aspect of the invention are essential features of the present invention.

It is also demonstrated in the examples that the method according to the first aspect of the invention bears advantages for the robust production of cells having a cardiomyocyte phenotype at larger scale. By the method of the invention cells having a cardiomyocyte phenotype can be conveniently initiated from routine stem cell cultures. The method of the invention is easier to control than prior art methods relying on a combination of various growth factors.

In accordance with a preferred embodiment of the first aspect of the invention the overexpression of EOMES comprises introducing into the ESCs or iPSCs a nucleic acid molecule encoding EOMES in an expressible form.

The term "nucleic acid molecule", in accordance with the present invention, includes DNA, such as cDNA or genomic DNA, and RNA. It is understood that the term "RNA" as used herein designates mRNA. The term "nucleic acid molecule" is interchangeably used in accordance with the invention with the term "polynucleotide".

The nucleic acid molecule encoding EOMES can be introduced into the ESCs or iPSCs by transformation. Methods for transformation of cells are well established in the state of the art and include but are not limited to viral transformation (e.g. adenoviral, adeno-associated, retroviral, lentiviral transfection), transposon transformation (e.g. retrotransposons, DNA-transposons, retroviruses as transposable elements, and in particular Tc1/mariner-class transposons like piggyBac and Sleeping Beauty), lipofection, microinjection, electroporation, impalefection, gene gun, magnetofectin, sono-poration, optical transfection (e.g. by a laser), and chemical-based transfection. Alternatively, the endogenous EOMES locus may be genetically modified to be activated at will - by any technically possible means - including, for instance, the insertion of an inducible regulatory element upstream of the natural EOMES transcription start site. The insertion of such an element may be triggered using CRISPR/Cas9, TALENs, or Zink-finger nucleases as accessory genetic tools.

In a more preferred embodiment of the method of the first aspect of the invention, the nucleic acid molecule encoding EOMES is a vector, preferably a transgenic vector.

Accordingly, the present invention uses a vector containing the nucleic acid molecule encoding EOMES. Preferably, the vector is a plasmid, cosmid, virus, bacteriophage or another vector used. conventionally in genetic engineering. A transgenic vector is a vector comprising a transgene. The term "transgene" designates a segment of DNA containing a gene sequence that has been isolated from one organism and is introduced into a different organism. In the present case the gene sequence encodes EOMES.

The nucleic acid molecule may be inserted into several commercially or publicly available vectors. The nucleic acid molecule may also be inserted into vectors such that a translational fusion with another polynucleotide is generated. The other polynucleotide may encode a protein which may e.g. increase the solubility, correct protein folding, facilitate the purifcation of the cells and/or further differentiation factors enhancing differentiation into cells having a cardiomyocyte phenotype. For vector modification techniques, see Sambrook and Russel (2001), Molecular Cloning: A Laboratory Manual, 3rd ed. Generally, vectors can contain one or more origin(s) of replication (ori) and inheritance systems for cloning or expression, one or more markers for selection in the host, e. g. dihydrofolate reductase, G418, neomycin, ampicillin, hygromycin, or kanamycin, and one or more expression cassettes. Suitable origins of replication (ori) include, for example, the Col E1, the SV40 viral and the M 13 origins of replication.

The coding sequences inserted in the vector can e.g. be synthesized by standard methods, or isolated from natural sources. Ligation of the coding sequences to transcriptional regulatory elements and/or to other amino acid encoding sequences can be carried out using established methods. Transcriptional regulatory elements (parts of an expression cassette) ensuring expression in ESCs and iPSCs are well known to those skilled in the art. These elements comprise regulatory sequences ensuring the initiation of transcription (e. g., translation initiation codon, promoters, such as naturally-associated or heterologous promoters and/or insulators), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers.

An expression vector used according to this invention is capable of directing the expression of the mRNA encoding EOMES. Suitable expression vectors which comprise the described regulatory elements are known in the art. The nucleic acid molecules as described herein above may be designed for direct introduction, phage vectors or viral vectors (e.g. adenoviral, retroviral) into a cell.

A typical mammalian expression vector contains the promoter element, which mediates the initiation of transcription of mRNA, the protein coding sequence, and signals required for the termination of transcription and polyadenylation of the transcript. Moreover, a mammalian expression vector may comprise further elements such as origin of replication, drug resistance gene, regulators (as part of an inducible promoter). Preferably the mammalian expression vector contains a regulator, more preferably an inducible promoter which allows for the induction of the overexpression of EOMES.

The activity of inducible promoters is induced by the presence or absence of biotic or abiotic factors. Inducible promoters are a very powerful tool in genetic engineering because the expression of genes operably linked to them can be turned on or off, as needed. There are virtually hundreds of inducible promoters that vary according to the organism source and cells or tissues where they regulate gene transcription. Examples of chemically-regulated promoters are promoters that can be regulated by alcohol, tetracycline, a steroid or a metal. Examples of physically-regulated promoters are promoters that can be regulated by temperature or light.

Highly efficient transcription can be achieved with the early and late promoters from SV40, the long terminal repeats (LTRs) from retroviruses, e.g., RSV, HTLVI, HIVI, the early promoter of the cytomegalovirus (CMV), or from selected mammalian housekeeping gene promoters such as pPGK. Alternatively, the recombinant polypeptide can be expressed in stable cell lines that contain the gene construct integrated into a chromosome. The co-transfection with a selectable marker such as dhfr, gpt, neomycin, hygromycin allows the identification and isolation of the transfected cells. The transfected nucleic acid can also be amplified to express large amounts of the encoded polypeptide.

In an even more preferred embodiment the vector is a lentiviral or transposon vector.

Examples of lentiviruses providing elements of a lentiviral vector are HIV, SIV, FIV, Puma lentivirus, EIA, Bovine immunodeficiency virus, Caprine arthritis encephalitis virus, or Visna/maedi virus. Preferably used in accordance with the invention is a 3rd generation lentiviral expression plasmid containing a SFFV promoter driven IRES-Puro (Foxa2), IRES-dTomato (C/EBPα) or IRES-eGFP (HNF4α) expression cassette. Lentiviruses are particularly useful in the method for the invention because they integrate into the genome and thus allow for non-transient gene expression. Moreover, studies have shown that lentivirus vectors have a lower tendency to integrate in places that potentially cause cancer than gamma-retroviral vectors which also integrate into the genome (Cattoglio et al. (2007), Blood, 110(6):1770-1778). Thus they have a lower tendency of abnormal cell growth upon integration.

Examples of transposon vectors are known in the art and comprise, for example, the PiggyBac (PB) transposon system or the Sleeping Beauty transposon system. The PiggyBac (PB) transposon is a mobile genetic element that efficiently transposes between vectors and chromosomes via a "cut and paste" mechanism. During transposition, the Super PB transposase recognizes transposon-specific inverted terminal repeat sequences (ITRs) located on both ends of the transposon vector and moves the contents from the original sites and efficiently integrates them into TTAA chromosomal sites. The powerful activity of the PiggyBac transposon system enables genes of interest between the two ITRs in the PB vector to be easily mobilized into target genomes. The Super PB transposase (is an engineered, hyperactive enzyme that catalyzes the integration of piggyBac transposons into TTAA sites in the target genome. The Sleeping Beauty transposon system is composed of a Sleeping Beauty (SB) transposase and a transposon that was designed to insert specific sequences of DNA into genomes of vertebrate animals. DNA transposons translocate from one DNA site to another in a simple, cut-and-paste manner. Transposition is a precise process in which a defined DNA segment is excised from one DNA molecule and moved to another site in the same or different DNA molecule or genome. The PiggyBac transposon system and the Sleeping Beauty transposon system can be combined with CRISPR/Cas9 to efficiently perform genetic manipulations in animal models or cells lines, including stem cells, without leaving behind any accessory DNA sequences.

Inducible lentiviral or transposon vectors allowing for the full expression control via inducible promoters are commercially available, for, example from Sirion Biotech or SBI. The lentiviral or transposon vector to be used is preferably a lentiviral or transposon vector wherein the overexpression is inducible, e.g. by placing the nucleic acid molecule encoding EOMES under the control of an inducible promoter.

In a preferred embodiment of the first aspect of the invention, EOMES is overexpressed via CRISPR-Cas genome editing.

CRISPR-Cas genome editing tools for inducing overexpression of a desired gene within a cell are commercially available; see, for example, https://www.addgene.org/crispr/activate/. Endogenous CRISPR systems fall into three categories, type I, II and III (Makarova et al., Nat Rev Microbiol. 2011 Jun; 9(6): 467-477.). Commercial CRISPR genome editing tools are adapted and often simplified from endogenous type II systems. Engineering of catalytically inactivated Cas variants (nuclease-deficient or nuclease-deactivated [dCas]) combined with transcriptional activators enable sequence-specific regulation of gene expression (Lo and Qi, Version 1. F1000Res. 2017; 6: F1000 Faculty Rev-747.). For instance, a catalytically inactive Cas9 (dCas9) fused to a transcription activator peptide can increase transcription of a desired gene, in the present case the gene encoding EOMES. For this purpose the guide RNA (gRNA) sequence has to be designed to direct the dCas9-activator to a specific genomic sequence. Potential target locations can include promoter regions and regulatory regions. If the chosen plasmid does not also express a gRNA, a separate gRNA expression plasmid is needed to target the dCas9-activator. Cas9 is an endonuclease that induces a double-strand break in genomic DNA, allowing the removal of genes or DNA sequences, or the integration of foreign DNA at specific sites. The gRNA has a scaffold sequence, which is needed for Cas9 binding, and a 20-nucleotide user-defined spacer or target DNA sequence. A Homologous Recombination (HR) template containing the nucleic acid molecule (e.g. encoding a transcriptional activator) to be introduced flanked by regions of homology is provided and introduced into the genome of the cell by the cellular DNA repair machinery.

Also inducible CRISPR-Cas genome editing tools exists. These systems are, for example, activatable by light (Nihongaki, Chemistry & Biology, (2015) 22(2):169-174). The CRISPR-Cas genome editing tool to be used is preferably an inducible CRISPR-Cas genome editing tool, because this allows for controlling the onset of EOMES overexpression in the cell.

In accordance with a further preferred embodiment of the first aspect of the invention, the pluripotent stem cells - embryonic stem cells or induced pluripotent stem cells - have been obtained from a subject or from a primary cell specimen.

As explained herein above, embryonic stem cells may be directly obtained from an embryo. Once embryonic stem cells were obtained they may also be used to establish an embryonic stem cell line. This has the advantage that no further embryos are needed and instead the embryonic stem cells of the cell line can be clonally expanded, as needed.

In the examples herein below cells from the HuES6 cell line are used. HuES6 is pluripotent human embryonic stem cell line with a normal 46XX karyotype. The stem cells were derived from human blastocysts and are strongly positive for a number of molecular markers of undifferentiated pluripotent human stem cells, including SSEA-3, SSEA-4, TRA-1-60, TRA-1-81, and alkaline phosphatase. Several pluripotent human embryonic stem cell lines are available (https://grants.nih.gov/stem_cells/registry/current.htm).

In accordance with a further preferred embodiment of the first aspect of the invention the WNT-pathway is inhibited by a compound selected from the group consisting of a small molecule inhibitor, a nucleotide-based inhibitor and an amino acid-based inhibitor.

A small molecule inhibitor is a low molecular weight organic compound which is by definition not a polymer. The small molecule of the invention is a molecule that binds with high affinity to it target molecule thereby inhibiting the WNT-pathway. The upper molecular weight limit for a small molecule is preferably 1500Da, more preferably 1000Da, even preferably 800Da and most preferably 500Da which allows for the possibility to rapidly diffuse across cell membranes, so that it can reach intracellular sites of action. Libraries of small organic molecules and high-throughput techniques for screening such libraries with a specific target molecule, in the present case a target molecule which results in the inhibition of the WNT-pathway are available in the art.

A nucleotide-based inhibitor comprises or consists of a nucleic acid sequence. The nucleic acid is preferably complementary to a nucleic acid sequence of at least 12 contiguous nucleotides of a target mRNA the silencing of which results in the inhibition of the WNT-pathway. The nucleotide-based inhibitor may comprise or consist of RNA, DNA or both. The nucleotide-based inhibitor of the invention is a molecule that binds specifically to its target mRNA. As used herein specific binding means that the inhibitor specifically targets the target mRNA and does substantially not exert any off target inhibitory effects, in particular on other cellular nucleic acid molecules.

An amino acid-based inhibitor comprises or consists of an amino acid sequence and preferably an amino acid sequence of at least 25, more preferably at least 50 amino acids. The amino acid-based inhibitor of the invention is a molecule that binds specifically to a target molecule (e.g. mRNA or protein) the inhibition of which results in the inhibition of the WNT-pathway. The amino acid-based inhibitor preferably comprises natural amino acids but may also comprise unnatural amino acids. The amino acid-based inhibitor is preferably selected or designed so that it specifically binds to its target molecule and displays essentially no off-target inhibition within the cell.

In accordance with a yet further preferred embodiment of the first aspect of the present invention, the nucleotide-based inhibitor is an aptamer, a ribozyme, a siRNA, a shRNA or an antisense oligonucleotide (such as a LNA-GapmeR, an Antagomir, or an antimiR) and the amino acid-based inhibitor is an aptamer, an antibody or a protein drug.

The term "aptamer" in accordance with the present invention refers to DNA or RNA molecules being either in the natural D-conformation or in the L-conformation ("spiegelmer") that have been selected from random pools based on their ability to bind other molecules. Aptamers have been selected which bind nucleic acid, proteins, small organic compounds, and even entire organisms. A database of aptamers is maintained at http://aptamer.icmb.utexas.edu/. More specifically, aptamers can be classified as DNA or RNA aptamers or peptide aptamers. Whereas the former consist of (usually short) strands of oligonucleotides, the latter consist of a short variable peptide domain, attached at both ends to a protein scaffold. Nucleic acid aptamers are nucleic acid species that have been engineered through repeated rounds of in vitro selection or equivalently, SELEX (systematic evolution of ligands by exponential enrichment) to bind to various molecular targets such as small molecules, proteins, nucleic acids, and even cells, tissues and organisms. The molecular target envisaged by the present invention is a molecule (e.g. a protein or mRNA) the inhibition of which results in the inhibition of the WNT pathway. Hence, aptamers can be produced against the desired target molecule. Aptamers offer the utility for biotechnological and therapeutic applications as they offer molecular recognition properties that rival those of the commonly used biomolecules, in particular antibodies. In addition to their discriminate recognition, aptamers offer advantages over antibodies as they can be engineered completely in a test tube, are readily produced by chemical synthesis, possess desirable storage properties, and elicit little or no immunogenicity in therapeutic applications. Non-modified aptamers are cleared rapidly from the bloodstream, with a half-life of minutes to hours, mainly due to nuclease degradation and clearance from the body by the kidneys, a result of the aptamer's inherently low molecular weight. Several modifications, such as 2'-fluorine-substituted pyrimidines, polyethylene glycol (PEG) linkage, etc. are available to scientists with which the half-life of aptamers easily can be increased to the day or even week time scale.

The term "ribozymes" refers to RNA molecules that act as enzymes in the absence of proteins. These RNA molecules act catalytically or autocatalytically and are capable of cleaving e.g. other RNAs or proteins at specific target sites but they have also been found to catalyze the aminotransferase activity of the ribosome. Selection of appropriate target sites and corresponding ribozymes can be done as described for example in Zaher and Unrau (2007), RNA, 13 (7): 1017-1026. Examples of well-characterized small self-cleaving RNAs are the hammerhead, hairpin, hepatitis delta virus, and in vitro-selected lead-dependent ribozymes. The organization of these small catalysts is in contrast to that of larger ribozymes, such as the group I intron. Meanwhile, the principle of catalytic self-cleavage has become well established. The hammerhead ribozymes are characterized best among the RNA molecules with ribozyme activity. Since it was shown that hammerhead structures can be integrated into heterologous RNA sequences and that ribozyme activity can thereby be transferred to these molecules, it appears that catalytic sequences for almost any target sequence can be created, provided the target sequence contains a potential matching cleavage site. The basic principle of constructing hammerhead ribozymes is as follows: An interesting region of the RNA, which contains the GUC (or CUC) triplet, is selected. Two oligonucleotide strands, each with 6 to 8 nucleotides, are taken and the catalytic hammerhead sequence is inserted between them. Molecules of this type were synthesized for numerous target sequences. They showed catalytic activity in vitro and in some cases also *in vivo.* The best results are usually obtained with short ribozymes and target sequences. Since the target sequence is in accordance with the present invention mRNA or protein, ribozymes being capable to specifically inhibiting the WNT pathway can be generated. For example, a ribozyme targeting the mRNA encoding Dishevelled or the protein Dishevelled may be used to inhibit the WNT pathway.

The aptamers and ribozymes may comprise modified nucleotides, such as locked nucleic acids (LNAs).

The term "antibody" as used in accordance with the present invention comprises, for example, polyclonal or monoclonal antibodies. Furthermore, also derivatives or fragments thereof, which still retain the binding specificity, are comprised in the term "antibody". Antibody fragments or derivatives comprise, inter alia, Fab or Fab' fragments, Fd, F(ab')2, Fv or scFv fragments, single domain VH or V-like domains, such as VhH or V-NAR-domains, as well as multimeric formats such as minibodies, diabodies, tribodies, tetrabodies or chemically conjugated Fab'-multimers (see, for example, Altshuler et al., Biochemistry (Mosc). 2010 Dec; 75(13):1584-605, Holliger and Hudson, Nat Biotechnol., 2005; 23(9):1126-36). The term "antibody" also includes embodiments such as chimeric (human constant domain, non-human variable domain), single chain and humanized (human antibody with the exception of non-human CDRs) antibodies. Various techniques for the production of antibodies and fragments thereof are well known in the art and described, e.g. in Altshuler et al., Biochemistry (Mosc). 2010 Dec; 75(13):1584-605. Thus, polyclonal antibodies can be obtained from the blood of an animal following immunisation with an antigen in mixture with additives and adjuvans and monoclonal antibodies can be produced by any technique which provides antibodies produced by continuous cell line cultures. Examples for such techniques are described, e.g. Harlow and Lane, Antibodies: A Laboratory Manual, Cold Spring Harbor Laboratory Press, 1988 and Harlow and Lane, Using Antibodies: A Laboratory Manual, Cold Spring Harbor Laboratory Press, 1999 and include the hybridoma technique originally described by Köhler and Milstein, Nature 256 (1975), 495-497, the trioma technique, the human B-cell hybridoma technique (e.g. Kozbor, Immunology Today 4 (1983), 72; Milstein, C (1999), BioEssays 21 (11): 966-73.) and the EBV-hybridoma technique to produce human monoclonal antibodies (Cole et al., Monoclonal Antibodies and Cancer Therapy, Alan R. Liss, Inc. (1985), 77-96). Furthermore, recombinant antibodies may be obtained from monoclonal antibodies or can be prepared de novo using various display methods such as phage, ribosomal, mRNA, or cell display. A suitable system for the expression of the recombinant (humanized) antibodies or fragments thereof may be selected from, for example, bacteria, yeast, insects, mammalian cell lines or transgenic animals or plants (see, e.g., US patent 6,080,560; Holliger and Hudson, Nat Biotechnol., 2005; 23(9):1126-36). Further, techniques described for the production of single chain antibodies (see, inter alia, US Patent 4,946,778) can be adapted to produce single chain antibodies specific for the target of this invention. Surface plasmon resonance as employed in the BIAcore system can be used to increase the efficiency of phage antibodies.

The term "protein drug" designates designer drugs that are derivatives of human proteins. These proteins are used as scaffold to create a protein drug by well-established screening procedures (see Tomlinson et al (2004), Nature Biotechnology, 22(5): 521-522). Non-limiting examples of human proteins which serve as a scaffold for designing protein drugs are transferrin, C-type lectins, trinectins, domain antibodies, kunitz domains, lipocalins and the Fyn SH3 domain.

The antisense technology for the downregulation of mRNA is well-established and widely used in the art to treat various diseases. The basic idea of the antisense technology is the use of oligonucleotides for silencing a selected target mRNA through the exquisite specificity of complementary-based pairing (Re, Ochsner J. 2000 Oct; 2(4): 233-236). Herein below details on the antisense construct compound classes of siRNAs, shRNAs and antisense oligonucleotides will be provided. As will be further detailed herein below, antisense oligonucleotides are single-stranded antisense constructs while siRNAs and shRNAs are double-stranded antisense constructs with one strand comprising an antisense oligonucleotide sequence (i.e. the so-called antisense strand). All these compound classes may be used to achieve downregulation or inhibition of a target mRNA.

In accordance with the present invention the target of antisense constructs in general as well as all the specific classes of antisense constructs being described herein is an mRNA the downregulation or inhibition of which results in the inhibition of the WNT pathway. It is a matter of routine in the field of antisense technology to design an antisense construct with a sufficient number of nucleotide matches to any target in order to ensure that no off targets become downregulated.

The term "siRNA" in accordance with the present invention refers to small interfering RNA, also known as short interfering RNA or silencing RNA. siRNAs are a class of 12 to 30, preferably 18 to 30, more preferably 20 to 25, and most preferred 21 to 23 or 21 nucleotide-long double-stranded RNA molecules that play a variety of roles in biology. Most notably, siRNA is involved in the RNA interference (RNAi) pathway where the siRNA interferes with the expression of a specific gene. In addition to their role in the RNAi pathway, siRNAs also act in RNAi-related pathways, e.g. as an antiviral mechanism or in shaping the chromatin structure of a genome. siRNAs have a well defined structure: a short double-strand of RNA (dsRNA), advantageously with at least one RNA strand having an overhang. Each strand typically has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells to bring about the specific knockdown of a gene of interest. Thus, any gene of which the sequence is known can in principle be targeted based on sequence complementarity with an appropriately tailored siRNA. The double-stranded RNA molecule or a metabolic processing product thereof is capable of mediating target-specific nucleic acid modifications, particularly RNA interference and/or DNA methylation. Also preferably at least one RNA strand has a 5'- and/or 3'-overhang. Preferably, one or both ends of the double-strand have a 3'-overhang from 1-5 nucleotides, more preferably from 1-3 nucleotides and most preferably 2 nucleotides. In general, any RNA molecule suitable to act as siRNA is envisioned in the present invention. The most efficient silencing was so far obtained with siRNA duplexes composed of 21-nt sense and 21-nt antisense strands, paired in a manner to have 2-nt 3'- overhangs. The sequence of the 2-nt 3' overhang makes a small contribution to the specificity of target recognition restricted to the unpaired nucleotide adjacent to the first base pair (Elbashir et al. Nature. 2001 May 24; 411(6836):494-8). 2'-deoxynucleotides in the 3' overhangs are as efficient as ribonucleotides, but are often cheaper to synthesize and probably more nuclease resistant. The siRNA according to the invention comprises an antisense strand which comprises or consists of a sequence which is with increasing preference complementary to at least 13 nucleotides, at least 14 nucleotides, at least 15 nucleotides, at least 16 nucleotides, at least 17 nucleotides, at least 18 nucleotides, at least 19 nucleotides, at least 20 nucleotides, or at least 21 nucleotides of an mRNA the silencing of which results in the inhibition of the WNT pathway.

A preferred example of a siRNA is an endoribonuclease-prepared siRNA (esiRNA). An esiRNA is a mixture of siRNA oligos resulting from cleavage of a long double-stranded RNA (dsRNA) with an endoribonuclease such as Escherichia coli RNase III or dicer. esiRNAs are an alternative concept to the usage of chemically synthesized siRNA for RNA interference (RNAi). For the generation of esiRNAs a cDNA of an mRNA template may be amplified by PCR and tagged with two bacteriophage-promoter sequences. RNA polymerase is then used to generate long double-stranded RNA that is complementary to the target-gene cDNA. This complementary RNA may be subsequently digested with RNase III from Escherichia coli to generate short overlapping fragments of siRNAs with a length between 18-25 base pairs. This complex mixture of short double-stranded RNAs is similar to the mixture generated by dicer cleavage *in vivo* and is therefore called endoribonuclease-prepared siRNA or short esiRNA. Hence, esiRNA are a heterogeneous mixture of siRNAs that all target the same mRNA sequence. esiRNAs lead to highly specific and effective gene silencing.

A "shRNA" in accordance with the present invention is a short hairpin RNA, which is a sequence of RNA that makes a (tight) hairpin turn that can also be used to silence gene expression via RNA interference. shRNA preferably utilizes the U6 promoter for its expression. The shRNA hairpin structure is cleaved by the cellular machinery into siRNA, which is then bound to the RNA-induced silencing complex (RISC). This complex binds to and cleaves mRNAs which match the shRNA that is bound to it. The shRNA according to the invention comprises an antisense strand which comprises or consists of a sequence which is with increasing preference complementary to at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides of an mRNA the silencing of which results in the inhibition of the WNT pathway.

The term "antisense oligonucleotide" in accordance with the present invention refers to a single-stranded nucleotide sequence being complementary by virtue of Watson-Crick base pair hybridization to an mRNA the silencing of which results in the inhibition of the WNT pathway. The antisense oligonucleotides may be unmodified or chemically modified. In general, they are relatively short (preferably between 13 and 25 nucleotides). Moreover, they are specific for target mRNA, i.e. they hybridize to a unique sequence in the total pool of targets present in the target cells. The antisense oligonucleotide according to the invention comprises or consists of a sequence which is with increasing preference complementary to at least 13 nucleotides, at least 14 nucleotides, at least 15 nucleotides, at least 16 nucleotides, at least 17 nucleotides, at least 18 nucleotides, at least 19 nucleotides, at least 20 nucleotides, at least 21 nucleotides, at least 22 nucleotides, at least 23 nucleotides, at least 24 nucleotides, or at least 25 nucleotides an of an mRNA the silencing of which results in the inhibition of the WNT pathway

The antisense oligonucleotide is preferably a LNA-GapmeR, an Antagomir, or an antimiR.

LNA-GapmeRs or simply GapmeRs are potent antisense oligonucleotides used for highly efficient inhibition of mRNA function. GapmeRs function by RNase H dependent degradation of complementary RNA targets. They are an excellent alternative to siRNA for knockdown of mRNA. They are advantageously taken up by cell without transfection reagents. GapmeRs contain a central stretch of DNA monomers flanked by blocks of LNAs. The GapmeRs are preferably 14-16 nucleotides in length and are optionally fully phosphorothioated. The DNA gap activates the RNAse H-mediated degradation of targeted RNAs and is also suitable to target transcripts directly in the nucleus. The LNA-GapmeR according to the invention comprises a sequence which is with increasing preference complementary to at least 13 nucleotides, at least 14 nucleotides, or at least 15 nucleotides of an mRNA the silencing of which results in the inhibition of the WNT pathway. The LNA-GapmeR technology is well established. LNA-GapmeRs are routinely designed using established algorithms. LNA-GapmeRs to a selected target are commercially available including positive and negative controls, for example, from Exiqon.

As mentioned, AntimiRs are oligonucleotide inhibitors that were initially designed to be complementary to a miRNA. AntimiRs against miRNAs have been used extensively as tools to gain understanding of specific miRNA functions and as potential therapeutics. As used herein, the AntimiRs are designed to be complementary to an mRNA the silencing of which results in the inhibition of the WNT pathway. AntimiRs are preferably 14 to 23 nucleotides in length. An AntimiR according to the invention more preferably comprises or consists of a sequence which is with increasing preference complementary to at least 15 nucleotides, at least 16 nucleotides, at least 17 nucleotides, at least 18 nucleotides, at least 19 nucleotides, at least 20 nucleotides, at least 21 nucleotides, at least 22 nucleotides, or at least 23 nucleotides of an mRNA the silencing of which results in the inhibition of the WNT pathway.

AntimiRs are preferably AntagomiRs. AntagomiRs are synthetic 2-O-methyl RNA oligonucleotides, preferably of 21 to 23 nucleotides which are preferably fully complementary to the selected target mRNA. While AntagomiRs were initially designed against miRNAs they may also be designed against mRNAs. The AntagomiRs according to the invention therefore preferably comprises a sequence being complementary to 21 to 23 nucleotides of an mRNA the silencing of which results in the inhibition of the WNT pathway. AntagomiRs are preferably synthesized with 2'-OMe modified bases (2'-hydroxyl of the ribose is replaced with a methoxy group), phosphorothioate (phosphodiester linkages are changed to phosphorothioates) on the first two and last four bases, and an addition of cholesterol motif at 3' end through a hydroxyprolinol modified linkage. The addition of 2'-OMe and phosphorothioate modifications improve the bio-stability whereas cholesterol conjugation enhances distribution and cell permeation of the AntagomiRs.

Antisense molecules (including antisense oligonucleotides, such as LNA-GapmeR, an Antagomir, an antimiR), siRNAs and shRNAs of the present invention are preferably chemically synthesized using a conventional nucleic acid synthesizer. Suppliers of nucleic acid sequence synthesis reagents include Proligo (Hamburg, Germany), Dharmacon Research (Lafayette, CO, USA), Pierce Chemical (part of Perbio Science, Rockford, IL, USA), Glen Research (Sterling, VA, USA), ChemGenes (Ashland, MA, USA), and Cruachem (Glasgow, UK).

The antisense molecules (including antisense oligonucleotides, such as LNA-GapmeR, an Antagomir, an antimiR), siRNAs, shRNAs may comprise modified nucleotides such as LNAs. The ribose moiety of an LNA nucleotide is modified with an extra bridge connecting the 2' oxygen and 4' carbon. The bridge "locks" the ribose in the 3'-endo (North) conformation, which is often found in the A-form duplexes. LNA nucleotides can be mixed with DNA or RNA residues in the oligonucleotide whenever desired. Such oligomers are synthesized chemically and are commercially available. The locked ribose conformation enhances base stacking and backbone pre-organization. This significantly increases the hybridization properties (melting temperature) of oligonucleotides.

In accordance with a more preferred embodiment of the first aspect of the present invention, the small molecule inhibitor is selected from Wnt-C-59, XAV-939, ICG-001, IWR-1-endo, LGK-974, LF3, CP21R7 (als0 known as CP21), NCB-0846, PNU-74654, Salinomycin, KYA1797K, IQ-1, PRI-724, KY02111, IWP-2, IWP-L6, FH535, WIKI4, SKL2001, Wnt agonist 1IWR-1, TNKS-656 and Compd 10.

As mentioned, Wnt-C-59 is used the examples herein below. The broad implications of Wnt/β-catenin signaling in development, in the adult body and in disease render the pathway a prime target for pharmacological research and development. For this reason next to Wnt-C-59 several further small molecule inhibitors of the WNT pathway are known and commercially available (Voronkov and Krauss, Curr Pharm Des. 2012 Feb; 19(4): 634-664). Non-limiting but preferred examples are XAV-939, ICG-001, IWR-1-endo, LGK-974, LF3, CP21R7 (also known as CP21), NCB-0846, PNU-74654, Salinomycin, KYA1797K, IQ-1, PRI-724, KY02111, IWP-2, IWP-L6, FH535, WIKI4, SKL2001, Wnt agonist 1IWR-1, TNKS-656 and Compd 10.

In accordance with an additional preferred embodiment of the first aspect of the present invention, the embryonic stem cells or induced pluripotent stem cells are EOMES tet-on cells and the overexpression of EOMES is induced in the embryonic stem cells or the induced pluripotent stem cells by tetracycline or a derivative thereof.

The concentration of the tetracycline or a derivative thereof is preferably in the range of 0.025 to 2.5 µg/ml, more preferably 0.1 to 1.0 µg/ml and most preferably 0.2 to 0.5 µg/ml.

As discussed herein above, the EOMES overexpression is preferably an inducible overexpression. A system for inducible expression being widely used in the state of the art is the Tet-on system. The Tet-on system has also been used in the examples herein below. While in a Tet-Off system, tTA is capable of binding the operator only if not bound to tetracycline or one of its derivatives, such as doxycycline, in a Tet-On system the rtTA protein is capable of binding the operator only if bound by a tetracycline or a derivative thereof. Thus, the introduction of tetracycline or a derivative thereof to the system initiates the transcription of the genetic product, being in the present case the mRNA encoding EOMES.

The tetracycline derivatives retain the capability of tetracycline of binding to the rtTA protein, thereby initiating the transcription of the mRNA encoding EOMES. Preferred examples of tetracycline derivatives are doxycycline, minocycline, metacycline, sancycline, chlorotetracycline, demeclocycline and tigecycline.

In accordance with a further preferred embodiment of the first aspect of the present invention, the embryonic stem cells or the induced pluripotent stem cells are EOMES knock-out cells.

Using EOMES knock-out cells safeguards against having any basal, naturally occurring back-ground expression level of EOMES in the ESCs and iPSCs. This advantageously allows for a more precise control the EOMES overexpression in the ESCs and iPSC and may help to prevent any unwanted differentiation of the ESCs and iPSCs. For this reason EOMES knock-out cells were employed in the examples.

Means and methods for the generation of knock-out cells are available in the art. In the examples the EOMES knock-out cells were generated by CRISPR/Cas-mediated genome editing. As shown in the examples, CRISPR/Cas-mediated genome editing is a simple method to selectively knock out EOMES expression from cells using gRNA clustered regularly interspaced short palindromic repeats/Cas9 endonucleases. Other examples are the Cre/loxP-system or the FLP-FRT recombination system.

In accordance with a yet further preferred embodiment of the first aspect of the present invention, the method further comprises 24h to 96h, preferably 48 to 72h after the overexpression of (a) the supplementation of retinoic acid (RA) or a synthetic agonist of the RA pathway for at least 24h, preferably for 24 to 72 hours.

RA is preferably used in the concentration range of 0.25 to 1uM. As illustrated in the examples, in the absence of RA the cells obtained by the method of the invention acquire the phenotype of ventricular cardiomyocytes, whereas by additional supplementation of RA the phenotype of arterial cardiomyocytes can be obtained.

Atrial and ventricular cardiomyocytes show some gene expression differences (e.g. of transcription factors, structural proteins, ion channels, etc.) and also display some functional differences. For example, they show different electrophysiological and contractile properties (Ng et al., Am J Physiol Cell Physiol. 2010; 299(6):C1234-49).

Within the context of the above preferred embodiment it is also possible to use an agonist of RA instead of or together with RA to induce an arterial cardiomyocytes phenotype. Non-limiting examples of RA agonists are BMS 493, BMS 453, EC 23, tretinoin, and tamibarotene.

In accordance with a further preferred embodiment of the first aspect of the present invention, the cells having a cardiomyocyte phenotype are characterized by one or more of the capability of synchronized beating, sarcomeric structural organization, self-contractile behavior, capability of force generation, the capability of generating action potentials, functional expression of cardiac ion channels, and the capability of electrical coupling with one another.

The above seven morphological properties characterize (atrial and ventricular) cardiomyocytes. It is preferred with increasing preference that the cells having a cardiomyocyte phenotype are characterized by at least two, at least three, at least four, at least five, at least six, and all of the seven morphological properties.

In accordance with another preferred embodiment of the first aspect of the present invention, the cells having a cardiomyocyte phenotype are characterized by the expression of at least one gene being selected from NKX2.5, TNNT2, TNNC1, TNNI, ACTC1, ACTN2, MYH6, MYH7, MYL3, MYL4, MYL7, MYL2, MYOM1, RYR2, CACNA1C, CKM, KCNH2, KCNQ1, KCNA5, SCN5A, IRX4, DHRS9 and NPPA.

The expression of the listed 23 genes determines an expression profile being specific for naturally occurring cardiomocytes, in particular human cardiomocytes. In this respect, it is of note that IRX4 and MYL2 are expression markers for ventricular cardiomocytes whereas KCNH2, DHRS9 and NPPA are expression markers for atrial cardiomocytes. Hence, these genes may serve for distinguishing atrial cardiomocytes from ventricular cardiomocytes.

All other expression markers characterize both, atrial and ventricular cardiomocytes. It is accordingly preferred with increasing preference that the cells having an atrial or a ventricular cardiomyocyte phenotype are characterized by the expression of at least 2, at least 3, at least 4, at least 5, at least 6, at least 7, at least 8, at least 9, at least 10, at least 11, at least 12, at least 13, at least 14, at least 15, at least 16, at least 17, and all 18 of the genes being selected from NKX2.5, TNNT2, TNNC1, TNNI, ACTC1, ACTN2, MYH6, MYH7, MYL3, MYL4, MYL7, MYOM1, RYR2, CACNA1C, CKM, KCNQ1, KCNA5 and SCN5A. It is preferred with increasing preference that the cells having an atrial cardiomyocyte phenotype are characterized by the expression of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, and all 21 of the genes being selected from NKX2.5, TNNT2, TNNC1, TNNI, ACTC1, ACTN2, MYH6, MYH7, MYL3, MYL4, MYL7, MYOM1, RYR2, CACNA1C, CKM, KCNQ1, KCNA5, SCN5A, KCNH2, DHRS9 and NPPA. Similarly, is preferred with increasing preference that the cells having a ventricular cardiomyocyte phenotype are characterized by the expression of at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, and all 20 of the genes being selected from NKX2.5, TNNT2, TNNC1, TNNI, ACTC1, ACTN2, MYH6, MYH7, MYL3, MYL4, MYL7, MYOM1, RYR2, CACNA1C, CKM, KCNQ1, KCNA5, SCN5A, IRX4 and MYL2.

In accordance with an additional preferred embodiment of the first aspect of the present invention, the cells having a cardiomyocyte phenotype are characterized by a significantly reduced expression of at least one gene being selected from CASQ2, MYOM3 and COX7A1 as compared to naturally occurring cardiomyocytes or cultured cardiomyocytes that were obtained from naturally occurring cardiomyocytes.

As discussed herein above, the cells having a cardiomyocyte phenotype closely resemble but are not identical to naturally occurring cardiomyocytes. In particular, the cells having a cardiomyocyte phenotype of the invention can be held distinct from naturally occurring cardiomyocytes by a significantly reduced expression of at least one gene being selected from CASQ2, MYOM3 and COX7A1. Means and methods to quantify and compare gene expression levels are described herein above, for example, qPCR. A significantly reduced expression is present when the expression is reduced to less than 10%, preferably less than 5% and most preferably less than 1% as compared to the expression level in naturally occurring cardiomyocytes (i.e. 100%).

In accordance with a further preferred embodiment of the first aspect of the present invention, the method further comprises isolating a single cell having a cardiomyocyte phenotype obtained by the method described herein above and optionally clonally expanding said cell.

The term "isolating" refers to the separation of a single cell from the initial culture dish and its transfer to a new culture vessel, such as for example a different cell culture dish or flask. Methods of isolation of cells are well known in the art and include, without being limiting, mechanical isolation, limited dilution as well as cell sorting approaches. Mechanical isolation relates to the selection and isolation of a cell by e.g. manual picking of the cell, where necessary under a microscope, automated picking by use of a robot or laser-capture micro-dissection. Manual picking of a cell may be performed by methods known in the art, such as for example aspiration of the cell into the tip of a pipette. Cell sorting approaches include, for example, magnetic activated cell sorting (MACS), flow cytometry activated cell sorting (FACS) or panning approaches. Such methods for cell isolation are well known in the art and have been described, e.g. in Dainiak et al., ((2007) Adv Biochem Eng Biotechnol. 2007;106:1-18), Murray ((2007) Acta Histochemica 109:171-176) or Tung et al. ((2007) Clin Lab Med. 27:453-468).

The term "expanding", in accordance with the present invention, refers to a multiplication of cells, thus resulting in an increase in the total number of cells. For example, cells can be expanded to at least 100 times their original number, such as at least 1.000 times their original number, preferably at least 10.000 times their original number, such as at least 100.000 times their original number etc.. Expansion of cells may be achieved by any known method, e.g. by culturing the cells under appropriate conditions to high density or confluence and subsequently splitting (or passaging) of the cells, as described herein above.

The term "clonally expanding" refers to the fact that one single cell is expanded to a plurality of cells, thus resulting in a cell culture of clones of the initial cell.

In accordance with a yet further preferred embodiment of the first aspect of the present invention, the cells having a cardiomyocyte phenotype obtained are free or substantially free of pathogens.

Pathogens to be avoided are well known to the skilled person and include, without being limiting, viruses such as for example Hepatitis virus A, B, C, Epstein-Barr-Virus or HIV-Virus and bacteria such as for example mycoplasm or chlamydia. The cells are considered to be essentially free of pathogens if no more than 1 in 10⁴ cells comprises pathogens, such as e.g. no more than 1 in 10⁶ cells, preferably no more than 1 in 10¹⁰ cells, such as e.g. no more than 1 in 10¹⁵ cells, such as no more than 1 in 10²⁰ cells, more preferably no more than 1 in 10³⁰ and most preferably no more than 1 in 10⁵⁰ cells.

The present invention relates in a second aspect to a cell obtained by the method of the first aspect of the invention.

To the inventors' best knowledge, such cells do not occur naturally. For the reasons discussed herein above, the cells having a cardiomyocyte phenotype closely resemble but are not identical to naturally occurring cardiomyocytes.

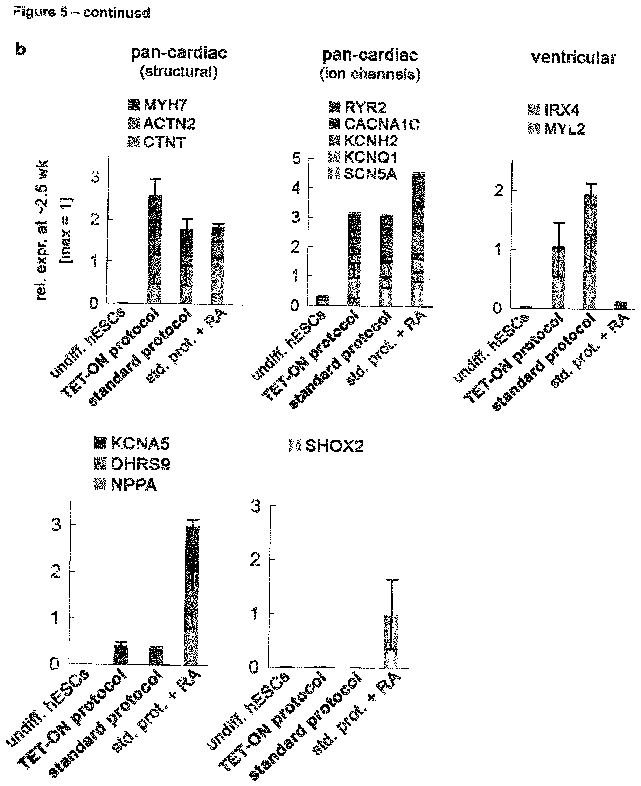

The cells obtained by the method of the first aspect of the invention are obtained by a method which involves the overexpression of a single gene and the inhibition of a single pathway. In contrast, the "classical" prior art methods a based on a cocktail of a number of growth factors that are to manipulate the cellular differentiation pathways so that cells having a cardiomyocyte phenotype are obtained. The cells having a cardiomyocyte phenotype of the present invention can be held distinct from cells having a cardiomyocyte phenotype that were obtained by the "classical" prior art method at least by a decreased expression of IRX4; see Fig 5b, third graph. Iroquois-class homeodomain protein IRX-4, also known as Iroquois homeobox protein 4, is a protein that in humans is encoded by the IRX4 gene. IRX-4 is important in early development but is not particularly important for the function of differentiated cardiomyocytes or cells having a cardiomyocyte phenotype. Hence, the reduced expression of IRX4 does not impair the function of the cells obtained by the method of the first aspect of the invention of resembling naturally-occurring cardiomyocytes, e.g., for any *in vivo* or *in vitro* applications. However, the decreased expression of IRX4 shows that the cardiomyocyte phenotype of the present invention can be distinguished from the phenotype of the cells that are obtained by the "classical" prior art method. Means and methods to quantify and compare gene expression levels of IRX4 are described herein above, for example, qPCR. A significantly reduced expression is present when the expression is reduced to less than 20%, preferably less than 10% and most preferably less than 5% as compared to the cells that are obtained by the "classical" prior art method (i.e. 100%).

The "classical" prior art method is preferably the "standard" protocol as used in the examples herein below. The "standard" protocol is a growth factor-based protocol, wherein the used growth factors are signaling agonists of the FGF, TGFβ, BMP, and WNT pathways, namely, a cocktail of the growth factors / small molecules FGF2, Activin A, BMP4, and CHIR99021 (a selective GSK3 inhibitor), respectively. This procedure resembles alternative published growth factor-based procedures which are likewise based on cocktails of signaling activators, mostly comprising a selection of the aforementioned molecules.

The present invention relates in a third aspect to the cell obtained by the method of the first aspect of the invention for use in medicine or medical or pharmaceutical research.

As shown in the examples of the application, the cells obtained by the method of the invention resemble naturally occurring cardiomyocytes. Thus, it can be expected that these cells can substitute or partly substitute naturally occurring non-functional or lost cardiomyocytes *in vivo,* in particular when used for the treatment or inhibition of a heart disease. Many heart diseases are associated with an impairment of the function of cardiomyocytes or even with a loss of cardiomyocytes, for example, due to apoptosis or programmed cell death. An example of a heart disease generally being associated with the impairment of the function of cardiomyocytes is cardiac arrhythmia. An example of a heart disease being associated with the loss of cardiomyocytes is heart failure.

Accordingly, the cell obtained by the method of the first aspect of the invention can be used in medicine for the treatment or prevention of a cardiac disease, wherein the cardiac disease is preferably selected from cardiac arrhythmia, heart failure, cardiac hypertrophy, myocardial infarction, and cardiomyopathy.

The cells having the phenotype of cardiomyocytes obtained by the method described herein are suitable for pharmacological or scientific research and may replace testing of cells directly obtained from the heart and/or animal experiments. In this regard compounds may be tested with regard to their effects to a cell having the phenotype of cardiomyocytes which has been obtained by the method of the invention. Such effects comprise pharmacological, cytotoxic, proliferative, transforming or differentiating effect. Such effects may, be for example, monitored by measuring cell proliferation, apoptosis, cell morphology, cell migration, gene expression, cellular protein, or metabolic processes.

Accordingly, the present invention relates in a fourth aspect to a method for identifying a compound having an pharmacological, cytotoxic, proliferative, transforming, differentiating, proarrhythmic, action potential modulating, calcium signaling modulating, electrical field potential modulating, or beating rate modulating effect on cells having a cardiomyocyte phenotype obtained by the method according to the first aspect of the invention, comprising: (a) contacting said cells having a cardiomyocyte phenotype with a test compound; and (b) determining whether the test compound has one or more of said effects on said cells having a cardiomyocyte phenotype.

In accordance with the present invention, the test compound may be selected from the group consisting of nucleic acids, such as DNA, cDNA, RNA, dsRNA, siRNA, shRNA, miRNA, proteins, peptides, sugars, lipids, small molecules (organic or inorganic), or any combination thereof.

Depending on the nature of the test compound, different step of contacting the cells may need to be adjusted. For example, if expressible proteins or peptides are to be evaluated, the corresponding coding sequences may be introduced as described herein above. In contrast, small molecules may be introduced by exogenously adding the respective compound to the cell medium and taking advantage of passive or active cellular uptake mechanisms. The skilled person is well-aware of methods of contacting cells with a test compound.

The function of test compound may be identified and/or verified by using high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the cell culture is preferably effected by one or more computer-controlled robotic systems including pipetting devices. Where large libraries of test compounds are to be screened and/or screening is to be effected within short time, mixtures of, for example 10, 20, 30, 40, 50 or 100 test compounds may be added to each well. In case a well exhibits an activity on the cells, said mixture of test compounds may be de-convoluted to identify the one or more test compounds in said mixture giving rise to the observed activity.

As described herein above, the cells having a cardiomyocyte phenotype of the invention are suitable for pharmacological or scientific research and may replace animal trials, testing on animal-derived cells, or testing of cardiomyocytes directly obtained from a subject. In this regard, compounds may be tested with regard to their effect on cardiomyocytes that have been obtained by the method of the invention. Such effects comprise pharmacological, cytotoxic, proliferative or anti-proliferative, transforming or differentiating effect. The skilled person is well aware of methods to assess whether a test compound has an effect on the cells having a cardiomyocyte phenotype of the invention, for example by comparing cells having a cardiomyocyte phenotype cultured in the presence and absence of the test compound. Depending on the effect to be assessed the methods vary and may include, e.g., visual control by microscopy, measuring cell proliferation, apoptosis, cell morphology, cell migration, gene expression, cellular or secreted protein levels, the presence of lipid mediators (for example prostaglandins or leukotriens) or metabolic processes.

For example, the cells of the present invention may be used to detect cardiotoxic side-effects of drugs developed in a non-cardiac context.

As another example, the cells of the present inventinon may be used to identify or validate novel drug as related to having beneficial effects on the function of cardiomyocytes. Furthermore, the underlying pluripotent stem cells may be genetically modified to yield models of a given cardiac disease. Alternatively, the cells may directly be derived from patient material. These disease models may subseqently be employed in drug screening paradigms to identify compounds / molecules having a disease-correcting effect according to any of the aforementioned assays.

As another example, the cells of the present invention may be used for cell therapeutic applications in which they would be used as a cellular agent in transplantation paradigms.

As another example, the process of programming pluripotent stem cells into cardiomyocytes based on the present invention may be used as a cellular assay for detecting negative effects of drugs on representative developmental processes such as cardiogenesis, as a model for human development during pregnancy.

As another example, the cells of the present invention or intermediates or the process may be used to identify novel means of manipulating cardiovascular cells in vitro or in vivo, for example to stabilize certain cell identities and promote their expansion, which includes, in particular, cardiovascular progenitor cells or the cells of the present invention themselves.

As another example, the cells of the present invention may be employed as biosensors to detect unwanted impurities in a composition for administration. Impurities that are toxic for humans, such as bacterial endotoxin (LPS), may thus be identified. Furthermore, the cells of the present invention may be employed to screen potential new inhibitors of signalling pathways triggered by pathogens, allergens or toxins.

As another example, the cells of the present invention can be employed to study the role of cardiomyocytes *in vivo.* Cells obtained in accordance with the present invention can for example be injected into an animal deficient for a certain function and the responses mediated by the cells of the present invention cells can be studied.

The above-discussed effects may be monitored using appropriate direct or indirect cellular assays related to one or more physiological, gene regulatory, or functional parameters. Examples of physiological and metabolic parameters include cell viability and cell death assays, assays for mitochondrial function, cell proliferation, structural organization, and metabolic activity using commercially available test kits or reagents, microscopic procedure, for instance. Examples of gene expression-based assays include quantification of gene expression levels using PCR-based methods, immunocytochemical detection of individual proteins, as well as reporter gene assays. Examples of functional readouts include fluorescence-based calcium imaging, fluorescence-based monitoring of action potentials, microelectrode array-based electrophysiological analysis, electrophysiological analysis using patch clamp methodologies, beating rate analysis using optical, electrophysiological, impedance, or mechanical read-outs, as well as structural and cell contraction analysis based on optical, mechanical, or impedance-based readouts. All these procedures may be conducted at the level of single cells or, alternatively, as tissue constructs or clusters or cells using appropriate tissue engineering and analysis platforms.

As regards the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

With respect to the following figures, figure legends and examples it is of note that the cells having a cardiomyocyte phenotype as obtained in accordance with the present invention are simply referred to as cardiomyocytes although they are - for the reasons as detailed herein above - not identical to naturally occurring cardiomyocytes.

The figures show.

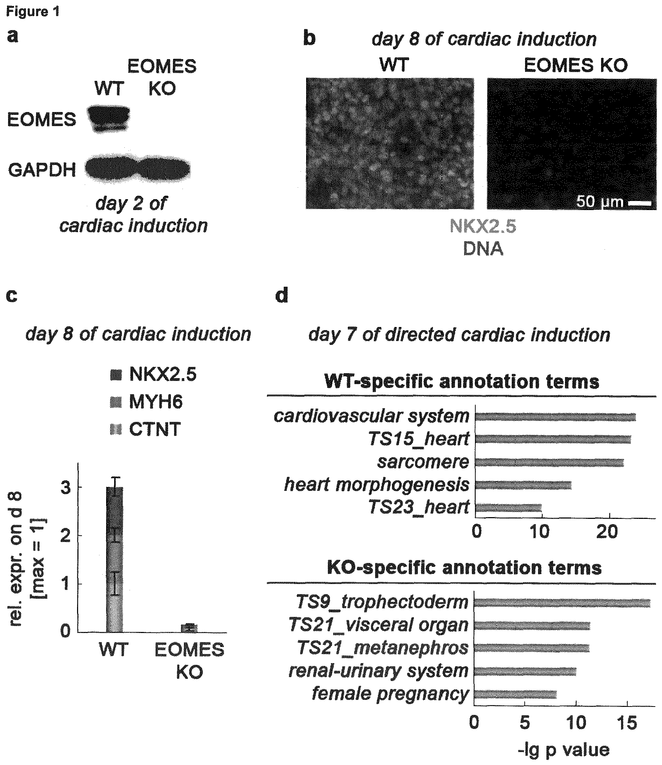

**Figure 1** EOMES knockout hESCs fail to differentiate into cardiomyocytes. **(a)** Immunoblot confirming EOMES expression and its absence in WT and KO cells, respectively, at the cardiac mesoderm stage of directed differentiation. **(b)** EOMES KO cells fail to express the early cardiomyocyte marker NKX2.5 following exposure to a directed differentiation protocol. **(c)** EOMES KO cells show a general failure in markedly upregulating essential pan-cardiac genes (qPCR data, n = 3). **(d)** Functional annotation of gene sets upregulated specifically in wild-type (top) or EOMES KO cells (bottom) following 7 days of differentiation with a directed cardiac induction protocol. The selected terms shown are based on stringent > 10-fold expression differences between d 7 samples as well as on comparisons against undifferentiated d 0 cells.

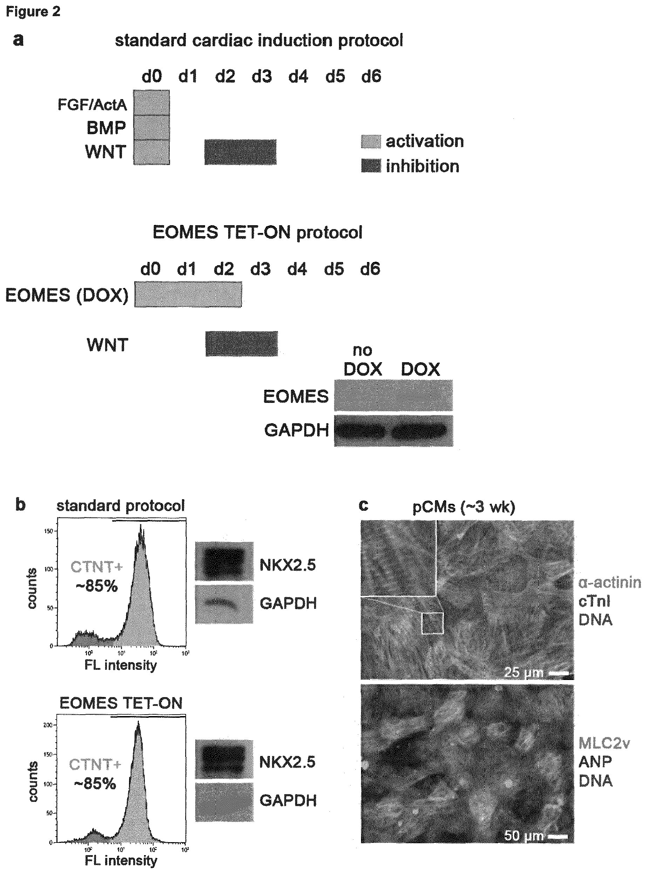

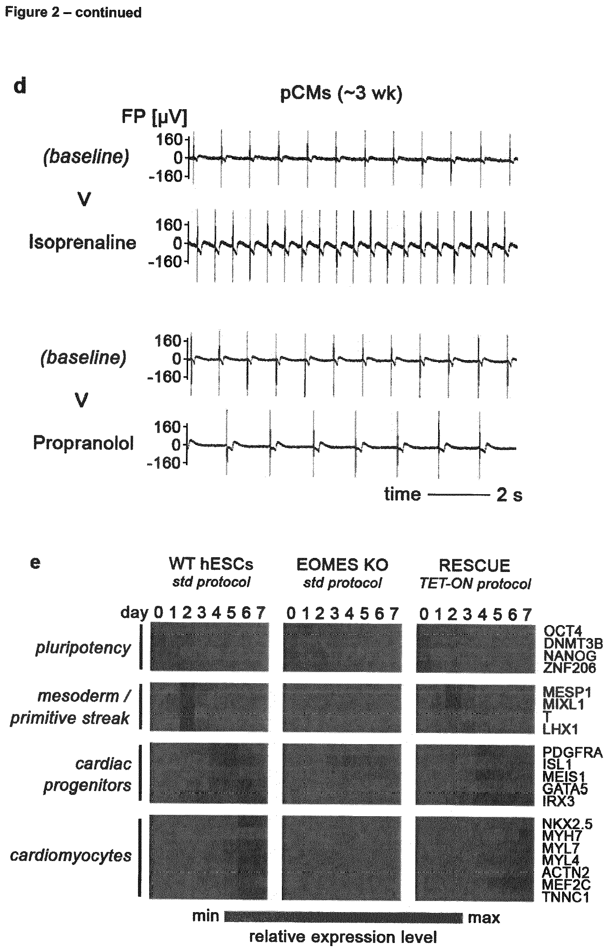

**Figure 2** EOMES programs hESCs into functional CMs at high efficiency. **(a)** Illustration of growth factor-mediated and optimized *EOMES* induction-based cardiac differentiation protocols. Bottom right: Immunoblot validating doxycycline-dependent EOMES expression in a transgenic EOMES^{KO/E.TET-ON} hESC line. **(b)** Typical yields of hESC-CMs (left, flow cytometry) and NKX2.5 expression (right, immunoblot) obtained with the two protocols after ∼1.5 wk. **(c)** Immunostainings ∼3 wk after the initiation of *EOMES* induction. Weak perinuclear ANP staining is typical in overall MLC2v-positive hESC-CMs. **(d)** Acceleration and slowdown of spontaneous beat rates in pCMs following exposure to 10 µM isoprenaline and 10 µM propranolol, respectively, on multielectrode arrays. **(e)** Microarray-based time-course analysis comparing the indicated protocols and cell lines. RESCUE cells carry an inducible *EOMES* transgene on EOMES^{KO} HuES6 background.

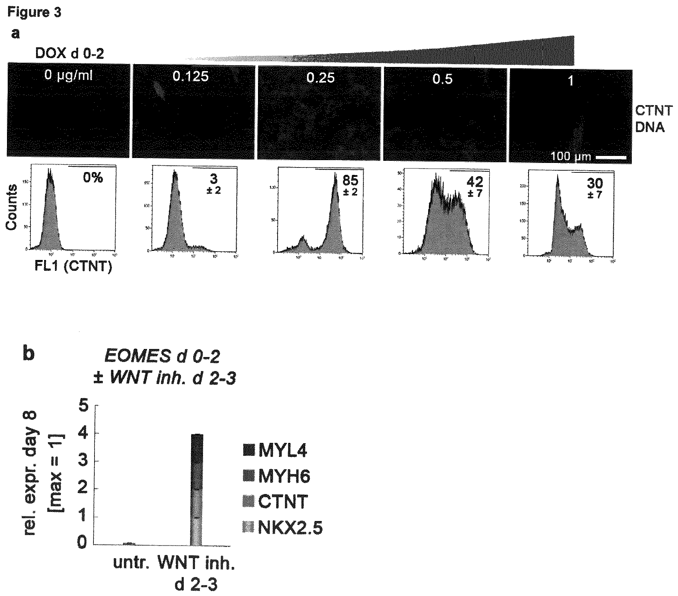

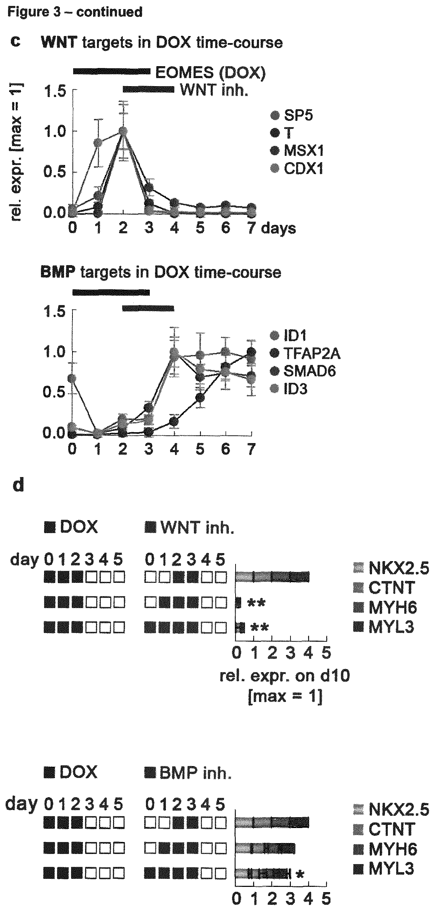

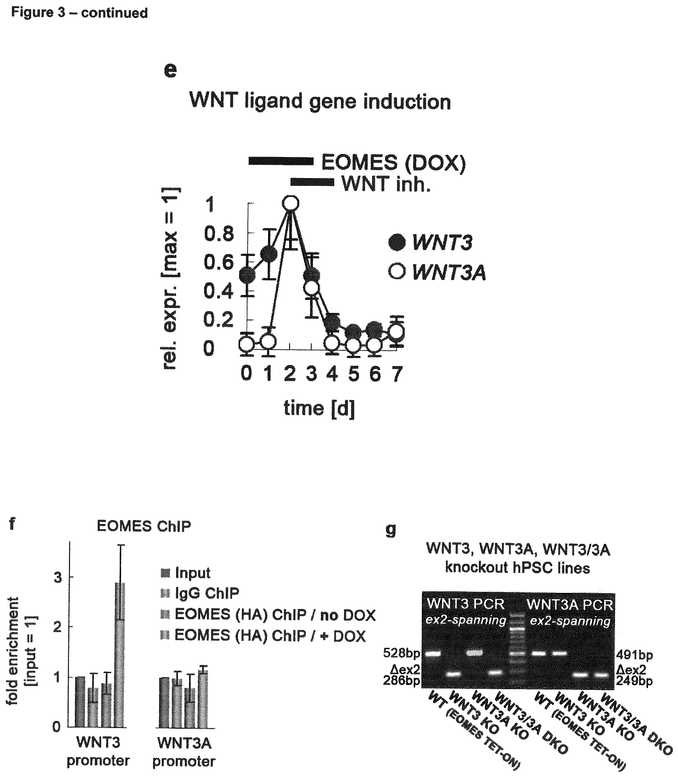

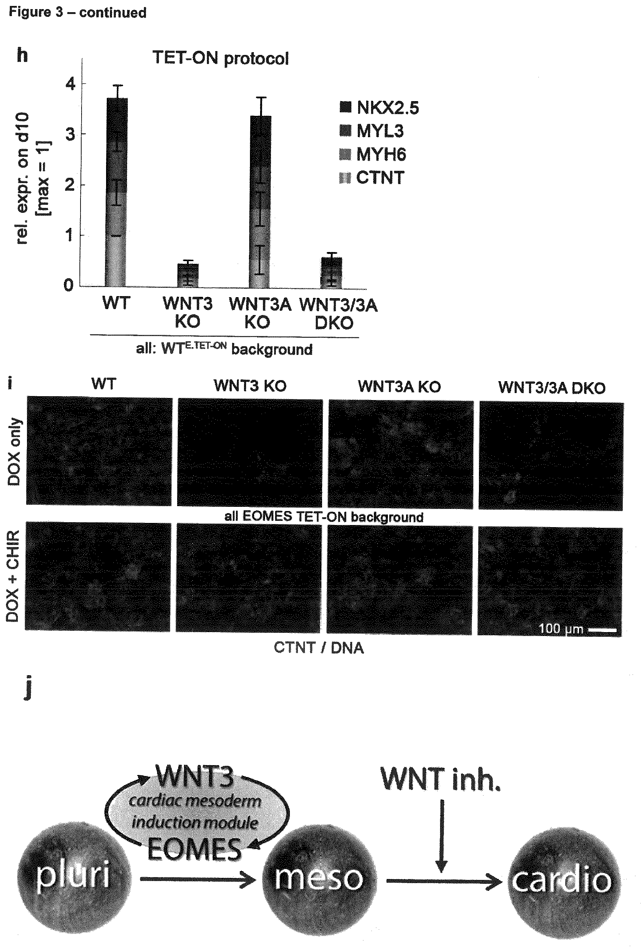

**Figure 3** Cardiac programming by EOMES is based on downstream induction of *WNT3.* **(a)** DOX dose dependency of the EOMES TET-ON protocol. Top panel: Immunostains at 1.5 wk. Bottom: Flow cytometry analysis. Numbers indicate average percentages of CTNT-positive CMs from 3-6 experiments per condition. **(b)** CM programming by EOMES necessitates suppression of autocrine WNT signaling from the third day of transgene induction (qPCR data, n = 2). **(c)** Short-term induction of canonical WNT but not BMP target genes during the time of DOX treatment. **(d)** Signaling inhibitor experiments with constant DOX from d 0-2 using indicated treatments with WNT (top) and BMP (bottom) antagonists (qPCR data, n = 3). WNT inhibitor C-59 was also administered to all samples of the bottom set to provide overall CM-permissive conditions as a baseline. **(e)** Canonical WNT ligand gene induction in the TET-ON protocol. **(f)** EOMES ChIP-qPCR using DOX-induced hESCs differentiated until the day 2 cardiac mesoderm stage (n = 3). Amplicons overlap with hit regions identified by ChlP-seq⁷. No-DOX samples served as specificity controls. **(g)** RT-PCR validation of frameshift-causing slice mutations induced by CRISPR/Cas9n in the *WNT3* and *WNT3A* genes. All cell lines were differentiated into cardiac mesoderm prior to RNA isolation. **(h)** TET-ON differentiation of the indicated cell lines (n = 3). **(i)** *WNT3* but not *WNT3A* is required for EOMES-mediated cardiac induction (top), and its discruption can be compensated by extrinsic WNT activation using CHIR99021 (bottom panel, representative immunostains at 2 wk). **(j)** General model illustrating the interplay between EOMES and WNT signaling in cardiac mesoderm induction. Subsequent WNT inhibition silences the module to allow differentiation to proceed.

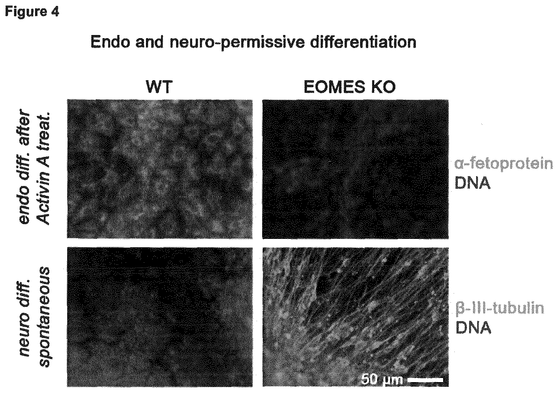

**Figure 4** Endoderm and neuroectoderm-permissive differentiation of WT and EOMES KO hESCs. Upper panel: EOMES KO cells fail to give rise to α-fetoprotein-expressing endoderm derivatives following an Activin A-assisted protocol. Bottom: EOMES KO cells display accelerated differentiation into neurons following spontaneous differentiation as embryoid bodies. WT cells did not yet show neuronal outgrowths at this relatively early time-point of analysis (∼2 wk).

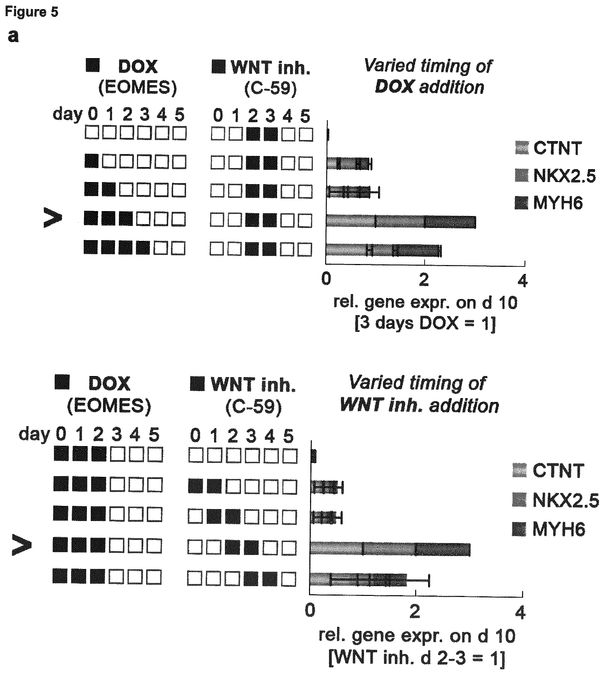

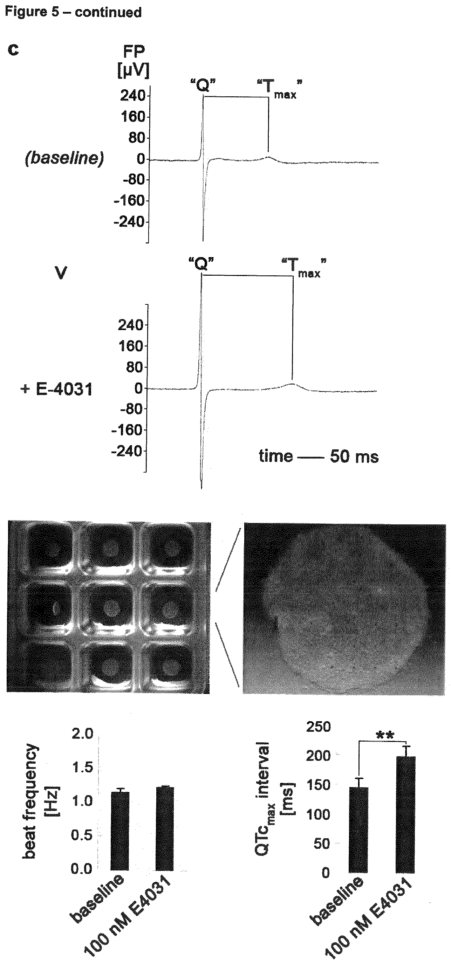

**Figure 5** Optimised generation and characterisation of DOX-induced cardiomyocytes. **(a)** Optimisation of parameters in the TET-ON protocol. Left: Variation time of exposure to doxycycline (qPCR data, n = 3-5 per condition). Right: Sliding window experiment to optimise the timing of the WNT signaling inhibition step (n = 2-5). Arrow heads indicate the best conditions subsequently used throughout. **(b)** Cardiac marker expression comparison between CMs generated with the TET-ON protocol, or with the standard growth factor-based protocol, or using additional retinoic acid (RA) supplementation to promote an atrial CM fate (qPCR data, n = 2-4 per data point, all HuES6 background). **(c)** Multielectrode array-based validation of hERG potassium channel functionality in pCMs. Top right: Electrode chip design with patches of beating pCMs. Left and bottom: hERG channel blocker E-4031 (100 nM) shifts indicated T wave-like signal in electrical field potential recordings to a later time-point, without significantly affecting spontaneous beating rates (n = 4).

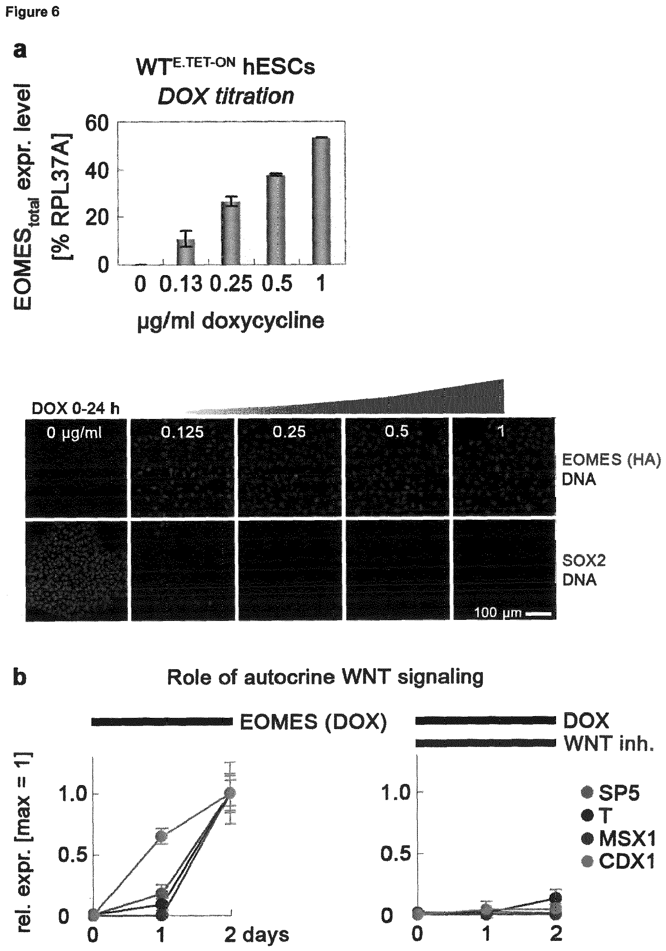

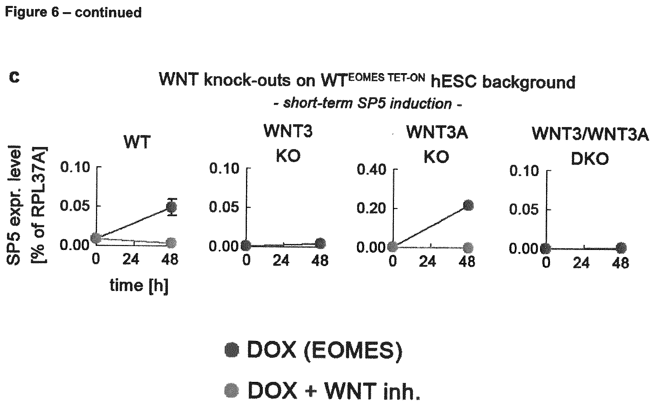

**Figure 6** Implication of WNT signaling in EOMES-driven cardiac induction. **(a)** Validation of a clonal EOMES TET-ON line generated on wild-type HuES6 background. DOX titration shows dose-dependent transgene induction and concomitant SOX2 repression as a key downstream effect (analysed at 48 h, n = 2). **(b)** DOX-mediated EOMES expression induces known WNT target genes within 2 days (left) and this effect is suppressed in the presence of a WNT inhibitor (C-59) that antagonises the secretion of WNT ligands (right; n = 3, normalised to no-WNTi d 2). **(c)** Early DOX-mediated induction of *SP5* is dependent on the ligand-encoding *WNT3* gene but not on *WNT3A,* as revealed by the lack of induction in WNT3 knockout and WNT3/WNT3A double-knockout cells under DOX (qPCR data, n = 2). WNT inhibitor-treated samples reconfirm *SP5* as a WNT target gene.

The examples illustrate the invention.

### Example 1 - Material and Methods

**hESC culture.** HuES6³¹ hESCs and its derivatives were maintained on 6-well dishes coated with 1 ml/well 1:75 diluted Matrigel™ HC (Corning # 354263), in defined FTDA medium³². FTDA was composed of DMEM/F12, 1 x PenStrep/Glutamine, 1 x defined lipids (Thermo), 1 x ITS (Corning), 0.1 % human serum albumin (Biological Industries), 10 ng/ml FGF2 (PeproTech # 100-18B), 0.2 ng/ml TGFβ1 (eBioscience # 34-8348-82), 50nM Dorsomorphin (Santa Cruz), and 5 ng/ml Activin A (eBioscience # 34-8993-85). Fully confluent hPSC cultures were harvested by a 15 min incubation with Accutase™ (Sigma) containing 10 µM ROCK inhibitor Y-27632 (abcamBiochemicals) and seeded out for passaging into new 6-well plates at 400,000 cells per well, in FTDA + ROCKi. Cells were split every 3-4 days and kept in culture for a maximum of 30 passages. Short-term signaling stimulation experiments were carried out using semiconfluent cultures.

**Genetic manipulation.** For the induction of splice mutations, two pairs of CRISPR/Cas9 nickase vectors were designed to encompass intron-exon boundaries. CRISPR vectors were generated by oligonucleotide cloning as described¹⁹, using the pX335 vector³³ (Addgene plasmid # 42335) modified to contain a GFP-2A-puromycin selection cassette. gRNA-specific targeting sequences are given in Table 1.

**Table 1 - gRNA-specific targeting sequences**

| **Gene** | **Product size (bp)** | **Fwd primer** | **Rev primer** |
|---|---|---|---|
| **Primers for RT-qPCR** | | | |
| ACTN2 | 75 | GCCAGAGAGAAGGATGCAATCAC | AAGCATGGGAACCTGGAATCAA |
| CACNA1C | 119 | CCAGGCTCCACGACTTCACA | GGCCTTTCTCGAGGGTGAGA |
| CDX1 | 86 | TGAGGAGGGAGGAACGTGGT | GGGCTCAGTGCCCTTATGATG |
| CTNT | 87 | GGCAGCTCCTGTTTGGAAATG | TTATTACTGGTGTGGAGTGGGTGTG |
| DHRS9 | 119 | TCCGGTGGTAGAGTGCATGG | GCAAAGCTGCTGGCATGTG |
| EOMES CDS | 76 | CGGCCTCTGTGGCTCAAA | AAGGAAACATGCGCCTGC |
| EOMES UTR | 71 | CTTGCTAGGCCTCTGCTGTGTG | TTGGTGACTCCTTAGCTTGCTCTCT |
| IRX4 | 77 | CGGAGCAGAAGAGGCCAGAT | CAGAACGGAACCGCCTTCTC |
| KCNA5 | 86 | CCAGCAGAGGGATAACCCAAAC | TTGGAACACATGGATGGAGGAG |
| KCNH2 | 115 | CGGTGCATGTGTGGTCTTGA | TGACATCTGCCTGCACCTGA |
| KCNQ1 | 113 | GCAGCCAGCCAAACACACA | GCGATGTAATGCCCAGAAGGA |
| MSX1 | 71 | AACCCTCACACTGCTCCAGTTTC | TTTGGCAGGGATCAGACTTCG |
| MYH6 | 101 | ACCTGGTGGACAAGCTGCAA | CACCTTGCGGAACTTGGACA |
| MYH7 | 74 | TTGATCTGCTCAGCCCTGGA | GCTTCCTCCCAAGGAGCTGTT |
| MYL2/MLC2v | 73 | TGGTCCCTGCCCTCATCTCT | GGCAGCCACATGGCTAACAG |
| MYL3 | 87 | AAGTTGATGGCTGGGCAAGA | GCACGAGGTTTAGCTGGACA |
| MYL4 | 87 | CTGGGCAAGAGGATGCCAAT | GCACCTGGAAGACTCTGCTTCA |
| NKX2-5 | 96 | ACCGATCCCACCTCAACAGC | CTCCGCAGGAGTGAATGCAA |
| NPPA / ANP | 77 | GCTGCAGCTTCCTGTCAACACT | AGGCGAGGAAGTCACCATCAA |
| RPL37A | 84 | GTGGTTCCTGCATGAAGACAGTG | TTCTGATGGCGGACTTTACCG |
| RYR2 | 83 | GGAGCCAGTGTCATCCACCA | CAGGTGGCTGAAAGAATGAGCA |
| SCN5A | 77 | ACTGCACAATGACCAGCAGGA | GTGAGAAGTGCTCGATTAGTTCAGACA |
| SHOX2 | 119 | TCTGTATCCCAATTCGCTAGCAA | CAACCGCATACCAAAGTTCAGTT |
| SP5 | 104 | TTCCAACTTCCGCTGCCTTC | CGTACGGCAGAGCTCCCAAT |
| T | 79 | CCTTGCTCACACCTGCAGTAGC | GGCCAACTGCATCATCTCCA |
| | | | |

| **Oligonucleotides for CRISPR gRNAs** | | | |
|---|---|---|---|
| WNT3 1/1' | n/a | GGCTCACAGCCCCTGCTCTG | GAGATGTGTACTGCTGGCCC |
| WNT3 2/2' | n/a | GAGGGTTCACAAAGCGGCCA | GGGGTAGGTGGAGAGGCAGA |
| WNT3A 1/1' | n/a | GCTACAGACCCTTTGCTCTG | GCTCATCTGTGTCGCTGTCC |
| WNT3A 2/2' | n/a | GATGGGCTGCGAGCCCAGGG | GTGTGCCAGCATCCCGGGCC |
| | | | |

| **RT-PCR primers for WNT3/3A knockout validation** | | | |
|---|---|---|---|
| WNT3 span ex2 | 528 wt / 286 mutant | CAGGGTCCTCGCTGGCTA | GCCTCGTTGTTGTGCTTGTT |
| WNT3A span | | | |
| ex2 | 491 wt / 249 mutant | GCCCCACTCGGATACTTCTT | ACCATCCCACCAAACTCGAT |
| WNT3 in ex2 | 486 wt / absent in mutant | GGGCCAGCAGTACACATCTC | GCCTCGTTGTTGTGCTTGTT |
| WNT3A in ex2 | 404 wt / absent in mutant | CAGTATTCCTCCCTGGGC | ACCATCCCACCAAACTCGAT |
| | | | |

| **ChIP primers** | | | |
|---|---|---|---|
| WNT3 promoter | 74 | GGACGGAGCCGAGTGTCATT | ATGCAAAGGCAGCAGGAGGT |
| WNT3A promoter | 76 | CACACCACGGATGAGTCTGGA | ACCGCGTTGGAATTGAGGAA |
| OTX2 | 106 | TCTGCCTTTGTCTTGGGATGC | GCCCATTCTTCTGCCAATTCA |

For disrupting a given locus, hESCs were transfected with the four corresponding CRISPR vectors using Fugene ™ HD (Promega). For simultaneously disrupting two genes, hESCs were accordingly transfected with a cocktail of 8 plasmids. One day later, transfectants were enriched using transient puromycin selection for 1 d (0.5 µg/ml). Two days later, semiconfluent cultures were replated at clonal density. Half the cells from single emerging colonies were used for DNA isolation and PCR screening using primers given in Table 1. The remaining half-colonies from positive clones were expanded and validated by RT-PCR sequencing.
Clonal DOX-inducible overexpression lines were generated using PiggyBac transposition as described¹⁹. Three vectors containing PiggyBac-flanked inducible EOMES-HA-IRES-Venus, PiggyBac-flanked constitutive rTA-IRES-NEO, and constitutive transposase transgenes were co-transfected into hESCs using Fugene HD, at a DNA mass ratio of 10:1:3, respectively. Stable transgene-positive cells were selected using 50 µg/ml G418 and replated at low density. Emerging colonies were split in half and replated. One plate was test-induced for 1 day using doxycycline and if homogeneous green fluorescence was observed for a given clone, the mirror clone was picked, expanded, and characterized as appropriate.

**Differentiation.** Standard growth factor-mediated cardiac induction was performed under serum and albumin-free conditions as described⁸. Briefly, fully confluent hESC cultures were harvested using Accutase resuspended in d 0 differentiation medium and seeded out at 500,000 cells/well in Matrigel-coated 24-well plates (2 ml volume/well). D 0 differentiation medium consisted of KO-DMEM, 1 x ITS, 10 µM Y-27632, 1 x PenStrep/Glutamine, 10-20 ng/ml FGF2, 0 or 5 ng/ml Activin A, 0.5-1 ng/ml BMP4 (R&D # 314-BP-050), and 1 µM CHIR99021 (AxonMedchem # Axon 1386). From day 1 onwards, the basal differentiation medium consisted of KO-DMEM, 1 x TS (transferrin/selenium), 250 µM 2-phospho-ascorbate, and PenStrep/Glutamine. WNT inhibitor C-59 (Tocris # 5148) was added to the cultures from 48-96 h of differentiation at 0.2 µM. Optionally, for promoting an atrial fate, all-trans-retinoic acid (Sigma # R2625) was supplemented from 72-120 h of differentiation. Differentiation medium was changed on a daily basis.
For EOMES-driven differentiation, transgenic hESCs were either replated into Matrigel-coated 24-well plates in FTDA + ROCKi (1 M cells/well), to initiate differentiation the day after or, alternatively, differentiated directly from subconfluent (d 3) maintenance cultures in 6-well format. Basal differentiation medium was identical to that in the growth factor-based protocol (insulin on d 0, no insulin thereafter). DOX was administered for the first three days at 0.25 µg/ml and C-59 from d 2-3 at 0.2 µM unless stated otherwise.
For CM maturation, beating monolayers emerging at d 6-8 were dissociated using TrypLE Select (Thermo) on ∼d 10 and replated at a ratio of ∼1:4 using CM splitting medium which consisted of RPMI 1640 (Thermo), 1 x ITS, 0.1 % HSA, 250 µM phospho-ascorbate, 0.008 % thioglycerol, 1 x PenStrep/Glutamine, and 10 µM ROCKi. Next day, medium was replaced by CM maintenance medium consisting of KO-DMEM, 1 x ITS, 0.1 % (w/v) HSA, 1 x defined lipids, 250 µM phospho-ascorbate, 0.008 % thioglycerol, and PenStrep/Glutamine. 1.5-2 wk later, cultured CMs were used for downstream analyses as indicated.

**RT-qPCR.** RNA was isolated using NucleoSpin RNA kits with on-column DNase treatment (Machery Nagel). Reverse transcription was performed using M-MLV reverse transcriptase (Affymetrix # 78306) with oligo-dT₁₅ priming at 42°C. Real-time PCR was carried out using validated primers given in Table 1 and BioRad iTaq™ Universal SYBR Green Supermix on ABI instrumentation. The ΔΔCt method was used to calculate relative transcript abundance against an indicated reference sample. Alternatively, results were expressed relative to a housekeeeping gene standard (*RPL37A,* 2^{-Δct}). Statistics were based on *RPL37A*-corrected Ct values or fold expression changes, as appropriate. Conventional RT-PCRs were performed according to standard procedures.

**Genome-wide expression analysis.** Labelled cRNA was prepared from 500 ng DNA-free RNA samples using TotalPrep™ linear RNA amplification kits (Thermo # AMIL1791). Microarray hybridizations on Illumina V4 human HT-12 bead arrays were carried out as recommended by the manufacturer. Cy3-stained chips were scanned using HiScan SQ instrumentation. Background subtraction and cubic spline normalization was done using GenomeStudio software. Processed data were filtered in MS Excel by setting experience-based thresholds for expression changes and minimal gene expression levels. For functional annotation of filtered gene lists, employing the Ensembl BioMart interface, array probe sequences were converted into GRCh37/hg19 genome coordinates which were then used as input for GREAT analysis³⁴. Statistically significant hits were subjectively filtered for biological relevance and presented based on the obtained p values.

**ChIP-qPCR.** Chromatin immunoprecipitation was performed as described¹⁹, using samples differentiated until the cardiac mesoderm stage (d 2). HA tag-based ChIP (Santa Cruz # sc-805-X) was used due to the temporal unavailability of a ChIP-grade EOMES antibody. IgG control was Santa Cruz # sc-2027. qPCRs were performed using diluted ChIP and serial dilutions of input DNA. Fold enrichments over input were calculated following internal normalization to an irrelevant control locus. Primers are given in Table 1.

**Immunocytochemistry.** Immunofluorescence analysis was carried out according to standard procedures using secondary Alexa-488 or Alexa-568-conjugated antibodies and Hoechst for fluorescent staining. Primary antibodies were α-actinin (Sigma # A7811, 1:800), ANP (R&D # AF3366, 1:100), α-fetoprotein (Sigma A8452, 1:500), HA tag (Santa Cruz # sc-806-X, 1:200), MLC2v (ProteinTech Group # 10906-1-AP, 1:200), NKX2.5 (R&D # AF2444, 1:100), cardiac troponin I (CTNI, Santa Cruz # sc-15368, 1:200), cardiac troponin T (CTNT, Thermo # MS-295-P, 1:200), β-III-tubulin (Covance # MMS-435P, 1:500), and SOX2 (R&D # AF2018, 1:200).

**Immunoblotting.** Western blotting was performed according to standard procedures using peroxidase-conjugated secondary antibodies and SuperSignal® West Pico chemiluminescent substrate (Thermo). Primary antibodies were EOMES (Abcam # ab23345, 1:1000), GAPDH (Thermo # AM4300, 1:10,000), and NKX2.5 (R&D # AF2444, 1:100).

**Flow cytometry.** Flow cytometry was carried out following Accutase digestion of primary monolayers or replated CMs, fixation with 2 % formaldehyde, and blocking / antibody incubations in FACS buffer (0.5 % saponin / 5 % fetal calf serum in PBS). Antibodies used were CTNT (Thermo # MS-295-P, 1:200) and Alexa-488 anti-mouse (Thermo # A11001, 1:1000).

**Electrophysiological analysis.** For analysis of pCMs on microelectrode arrays (USB-MEA256 system, Multichannel Systems), the electrode areas of plasma-cleaned 9-well MEAs were coated with 3 µl of a 1:75 diluted Matrigel solution in KO-DMEM for approximately 2 hours at 37°C in a humidified cell culture incubator. pCMs were dissociated from maintenance cultures using a 10 x TrypLE Select digestion to obtain a single-cell / small aggregate suspension. Coating solution was removed from the electrode arrays to be replaced by 25,000-50,000 cells resuspended in a ∼3 µl droplet of CM splitting medium. CMs were allowed to attach for ∼30 min. Subsequently, MEA chambers with attached cells were filled with 150 µl of CM replating medium. Next day, medium was changed to CM maintenance medium. From the following day onwards, cell preparations were used for recordings at 37°C. Drugs were washed in for 5 min (isoprenaline: 10 µM, propranolol: 10 µM, E-4031: 100 nM). Algorithms for QTₘₐₓ detection were implemented in MC Rack software v4.5.7. Field potential durations (QTₘₐₓ-like intervals) were averaged from independent replicates. Values were frequency-corrected using Bazett's formula: QTcₘₐₓ = QTₘₐₓ [ms] / (RR [s])^{0.5}.

**Statistics and reproducibility.** Essentially every result was confirmed in at least one additional experiment. The TET-ON protocol has independently been reproduced multiple times by three individuals, with highly similar outcomes. Unless stated otherwise, numerical data represent means of n biological replicates, as indicated in figure legends. Error bars of qPCR data denote s.e.m., and those of microarray data reflect bead standard deviation. Where meaningful, 1 or 2-sided unpaired t-tests were used, as appropriate, to compare pairs of samples in MS Excel. * denotes a significance level of p < 0.05, and ** < 0.01. Statistics on microarray data were based on an Illumina custom model implemented in GenomeStudio software.

### Example 2 - Results and Discussion

Essentially all heart cells are descendants of EOMES-expressing cells in mouse development^{3, 9, 10}. Similarly, most cells forming the heart are derived from mesoderm precursors expressing the bHLH transcription factor MESP1^{11, 12}. MESP1 has been proposed to play a master regulatory role in cardiovascular specification¹³. This view is based on procardiac effects observed in elegant MESP1 gain-of-function studies using mouse ES cells and vertebrate embryos¹³⁻¹⁶. *Mesp1* is a target gene of EOMES and hence it is thought that EOMES exerts its cardiogenic function through this mechanism^{3, 6}. However, neither EOMES nor MESP1-expressing cells in the embryo exclusively give rise to the cardiac lineage, since both genes also play prominent roles in other contexts^{4, 9, 16}. Accordingly, overexpression studies in mouse ES cells have thus far yielded rather moderate cardiogenic effects over background^{6, 13-16}. Therefore, the issue of whether there is a *bona fide* master regulatory factor specifically promoting the induction of cardiac cells at high efficiency, and under which conditions it would do so, appears to be unresolved.

Human ES cells (hESCs) present an excellent model system to investigate such questions. This is because controlled differentiation procedures, including directed cardiac induction protocols, are in part highly developed by now and these are based on developmental principles^{17, 18}. In addition, genetic manipulation tools have emerged that now permit systematic loss and gain-of-function studies, in combination with modifying the extrinsic signaling environment at high temporal resolution.

Following up on previous investigation of cardiac induction mechanisms in the hESC system¹⁹, EOMES knockout (KO) hESCs were subjected to a stringent cardiac differentiation protocol⁸. At the cardiac mesoderm stage of this procedure, day 2²⁰, EOMES was confirmed to be highly expressed in wild-type (WT) cells and absent in KO ones (Fig. 1 a). At day 8, WT cells had formed beating monolayers expressing the early cardiomyocte marker NKX2.5 and other pan-cardiac genes, whereas EOMES KO cells did virtually not express any of these (Fig. 1b,c). Genome-wide expression profiling revealed gene sets specifically upregulated in WT and KO cells towards the endpoint of the protocol. As expected, WT cells were restricted to the cardiac lineage. By contrast, the cell fate of KO cells was surprisingly characterised by extraembryonic and, interestingly, renal differentiation-associated terms and marker genes such as *KANK4*²¹ (Fig. 1d). Moreover, using low-stringency differentiation conditions permissive for endodermal or neural fates, it was found that EOMES KO cells were defective in the former but highly competent for the latter (Fig. 4). These results confirm the essential roles of EOMES in endoderm and cardiac specification while indicating preserved competence of KO hESCs for differentiation into alternative fates, including non-cardiac mesoderm derivatives.

Given the severe failure of EOMES KO hESCs to form cardiomyocytes (CMs) under directed differentiation conditions, it was next asked whether enforced *EOMES* induction could in turn drive the process on its own. Using an inducible overexpression cell line on EOMES^{KO} background in which an EOMES transgene may be activated using doxycycline (DOX) administration (EOMES^{KO/E.TET-ON})¹⁹, the standard cardiac induction procedure was modified such that all cardiac mesoderm-inducing signaling factors were replaced by DOX (Fig. 2a). Optimization of the timing of DOX supplementation and that of a WNT inhibitor added in a subsequent stage revealed that a 3-day DOX combined with 2 days of WNT inhibition (from 48-96 h) was most optimal (Fig. 5a). Strikingly, this protocol - devoid of any signaling pathway-activating molecules - generated near-homogeneous monolayers of beating CMs, similar to the standard procedure (Fig. 2b).

The hESC-CMs programmed by EOMES were termed pCMs and characterized as follows. pCMs expressed structural markers and ion channel genes involved in forming cardiac action potentials, at similar levels compared to standard hESC-CMs (Fig. 5b). Furthermore, pCMs displayed robust staining for cardiac markers at the protein level and showed a striated sarcomeric pattern in many cells already by 3 wk (Fig. 2b,c). hESC-CMs tend to acquire an overall ventricular subtype identity by default, whereas an atrial fate may be induced by retinoic acid addition during early stages of differentiation²². This paradigm was adopted and employed to assess the subtype identity of pCMs. pCMs robustly expressed ventricular-specific myosin light chain 2 in almost all cells and were essentially deficient in atrial-specific ANP expression (Fig. 2c). These and other heart chamber-specific markers suggest that pCMs take on an overall ventricular identity, similar to standard hESC-CMs but different from retinoic acid-treated ones (Fig. 5b).

To verify this conclusion in a more functional way, pCMs were treated with E-4031, an inhibitor of the hERG potassium channel that plays a prominent role in ventricular repolarisation²³. Analysis on multielectrode arrays revealed that electrical field potential durations were significantly prolonged following E-4031 administration, which indicates that hERG is operative in pCMs (Fig. 5c). Furthermore, pCMs showed physiological responses to chronotropic drugs, namely, beat rate acceleration in response to isoprenaline, an adrenergic agonist, and slowdown after addition of propranolol which is a beta blocker (Fig. 2d). These data indicate that pCMs compare well with conventional hESC-CMs regarding their overall characteristics and acquired maturation features upon prolonged *in vitro* culture⁸.

It was next sought to better understand cardiac programming by EOMES. A time-course gene expression analysis revealed that EOMES KO cells not only failed to eventually upregulate a cardiac programme but that they were already lacking entire sets of mesodermal and primitive streak genes at early differentiation stages. However, *EOMES* induction via the TET-ON protocol fully restored the normal sequence of events (Fig. 2e). Hence, the similarity between conventional hESC-CMs and pCMs at later stages is matched by highly similar induction kinetics of mesodermal and cardiac gene expression in the short term.

To check whether the endogenous *EOMES* gene influences the efficacy of the transgenic induction system in a negative way, an EOMES TET-ON line on WT hESC background was additionally generated (Fig. 6a). There was no apparent difference in cardiac differentiation efficiencies and in general, the system conveniently allowed pCM induction from routine hESC maintenance plates simply by adding DOX to subconfluent 6-well cultures.

Both on WT and EOMES KO background, however, pCM induction efficiency was highly dependent on the DOX concentration used (Fig. 3a). This behaviour was reminiscent of the standard growth factor-based protocol in which BMP and/or WNT signaling agonists can easily be overdosed, giving rise to non-cardiac fates in such cases¹⁹. It was hence hypothesised that the profound impact of EOMES on global gene expression may in part be based on a link to WNT or BMP signaling. Indeed, the TET-ON protocol strictly required the inactivation of WNT signaling in a subsequent differentiation stage, possibly because the pathway becomes activated during the first two days of differentiation (Fig. 3b).

Short-term WNT and BMP stimulation experiments revealed pathway-specific target genes in hESCs. Some of the most stringent ones were then monitored in the TET-ON differentiation time-course. This analysis indicated that during the first two days of *EOMES* induction, target genes of canonical WNT signaling became strongly upregulated, whereas BMP-linked genes only showed a response thereafter (Fig. 3c). Notably, some of the early-induced WNT targets, like *MSX1* and *CDX1*/*2,* are known to be counterproductive for cardiac differentiation¹⁹. Their upregulation explains the necessity for subsequent WNT inhibition and argues for the involvement of a global - not necessarily cardiac-specific - WNT response. As expected, WNT inhibition during the first two days of DOX treatment prevented the upregulation of WNT targets including *T*(Brachyury), indicating that EOMES alone was not sufficient for their induction (Fig. 6b). Similar treatments also suppressed cardiac differentiation in the longer term, which demonstrates the functional importance of an initial activation of WNT signaling downstream of EOMES (Fig. 3d, top). In comparison, early BMP inhibition only had a mild effect (Fig. 3d, bottom).

To identify a possible mechanism for these observations, the TET-ON differentiation time-course data set was mined for the early induction of genes encoding canonical WNT signaling ligands. Two such genes, *WNT3* and *WNT3A,* were markedly upregulated by *EOMES* overexpression within 1-2 days. Both of these were also bound by EOMES in differentiating hESCs according to a previously published ChIP-seq data set⁷. Using ChIP-qPCR amplicons overlapping with the corresponding promoter regions, *WNT3 -* but not *WNT3A -* was confirmed as being a direct EOMES target gene in the protocol (Fig. 3f). Nonetheless, it was sought to investigate the functional implication of both ligand-encoding genes in an unbiased manner. Hence, homozygous knockout hESC lines for both *WNT3* and *WNT3A* were prepared using quadruple CRISPR/Cas9n DNA nicking²⁴. To account for a potential compensation between the two targeted genes, a *WNT3*/*WNT3A* double knockout line was also generated (DKO, see Example 1). These lines were prepared on WT^{E.TET-ON} background to later be able to challenge WNT ligand function using the TET-ON protocol. The induced splice mutations caused the predicted exon 2 deletions and associated reading frame shifts at RNA level, as evidenced by exon-spanning RT-PCR and cDNA sequencing (Fig. 3g).

DOX supplementation for two days induced *SP5*, a stringent WNT-specific target gene in hESCs, only in WT^{E.TET-ON} cells as well as in the WNT3A knockout line, but not in WNT3 KO or WNT3/WNT3A DKO cells (Fig. 6c). These data indicate that WNT3, not WNT3A, mediates the EOMES-induced WNT response early in the protocol. Furthermore, they predicted that CM formation would be compromised in the *WNT3* mutant lines. Indeed, cardiac differentiation efficiencies were unaffected in WNT3A KO cells but severely diminished in WNT3 KO and WNT3/WNT3A DKO ones (Fig. 3h). Finally, to formally demonstrate that this phenotype in WNT3-deficient cells was due to the specific disruption of the gene rather than unspecific effects, exogenous WNT activation to compensate for it was used. Indeed, an additional treatment with CHIR99021, a GSK3 inhibitor, rescued the genetic defect to restore cardiac differentation competence in the two WNT3-deficient lines (Fig. 3i).

Overall, these data reveal that the fundamental role of EOMES in germ layer induction^{4, 9} goes beyond activating individual factors downstream in the differentiation cascade, although the promotion of genes like *Mesp1* is certainly very important, too^{3, 6}. Rather, it is proposed that much of the function of EOMES is intimately based on a cooperative interplay with the WNT pathway, at least in cardiac mesoderm specification. Both EOMES and WNT are crucially required for cardiac induction. Under cardiac-permissive conditions, WNT signaling activates *EOMES*¹⁹ and EOMES promotes WNT signaling through the induction of *WNT3* as shown here. The two entities hence form a self-sustaining regulatory module in cardiac mesoderm specification (Fig. 3j). In this model, cardiac induction may either be accomplished through exogenous WNT activation as in WNT-driven cardiac differentiation protocols^{25, 26}, or by intrinsic *EOMES* induction as shown here - the module becomes activated in both ways. These mutual links between *EOMES* and *WNT3* are likely to have *in vivo* relevance as *Wnt3* is fundamentally required for mouse primitive streak formation whereas *Wnt3a* only plays a role at later stages^{27, 28}.

Overamplification of the module promotes non-cardiac fates. To prevent this from happening *in vivo,* migratory cardiac mesoderm cells assume residence at the anterior side of the embryo where they become exposed to WNT-antagonising factors, which will inactivate the regulatory circuit and counteract anti-cardiac gene expression^{19, 29}. In a cell culture setting, WNT or EOMES need to be dose-controlled to prevent the module from overshooting and additionally, sustained signaling is to be inhibited in a subsequent step (Fig. 3h). In this regard, EOMES serves as a context-dependent master regulator of cardiac specification. The fact that this key inductive function is highly dose and context-dependent may explain why it remained somewhat underestimated in a previous report using mouse ES cells⁶. Overall, the signaling context appears to have a profound impact on differentiation driven by intrinsic master regulators. For instance, activation of Nodal-SMAD2/3 signaling cooperates with EOMES to redirect cell fate along the endodermal lineage^{6, 7}. And even classical examples such as MYOD1-driven forward programming of hESCs into skeletal muscle requires concomitant retinoic acid signaling for optimal outcomes³⁰. Hence, environmental constraints do not question but may overshadow key roles of intrinsic regulators in cell programming.

From a practical point of view, EOMES-driven cardiac induction bears advantages for the robust production of CMs at a larger scale. pCMs display functional properties comparable to conventional hPSC-CMs but their generation could conveniently be initiated from routine cultures and are easier to control than growth factor-based differentiation.

### References

1. Whyte, W.A. et al. Master transcription factors and mediator establish super-enhancers at key cell identity genes. Cell 153, 307-319 (2013).
2. Tam, P.P. & Loebel, D.A. Gene function in mouse embryogenesis: get set for gastrulation. Nat Rev Genet 8, 368-381 (2007).
3. Costello, I. et al. The T-box transcription factor Eomesodermin acts upstream of Mesp1 to specify cardiac mesoderm during mouse gastrulation. Nat Cell Biol 13, 1084-1091 (2011).
4. Arnold, S.J., Hofmann, U.K., Bikoff, E.K. & Robertson, E.J. Pivotal roles for eomesodermin during axis formation, epithelium-to-mesenchyme transition and endoderm specification in the mouse. Development 135, 501-511 (2008).
5. Ryan, K., Garrett, N., Mitchell, A. & Gurdon, J.B. Eomesodermin, a key early gene in Xenopus mesoderm differentiation. Cell 87, 989-1000 (1996).
6. van den Ameele, J. et al. Eomesodermin induces Mesp1 expression and cardiac differentiation from embryonic stem cells in the absence of Activin. EMBO Rep 13, 355-362 (2012).
7. Teo, A.K. et al. Pluripotency factors regulate definitive endoderm specification through eomesodermin. Genes Dev 25, 238-250 (2011).
8. Zhang, M. et al. Universal Cardiac Induction of Human Pluripotent Stem Cells in 2D and 3D formats - Implications for In-Vitro Maturation. Stem Cells (2015).
9. Russ, A.P. et al. Eomesodermin is required for mouse trophoblast development and mesoderm formation. Nature 404, 95-99 (2000).
10. Ciruna, B.G. & Rossant, J. Expression of the T-box gene Eomesodermin during early mouse development. Mech Dev 81, 199-203 (1999).
11. Saga, Y., Kitajima, S. & Miyagawa-Tomita, S. Mesp1 expression is the earliest sign of cardiovascular development. Trends Cardiovasc Med 10, 345-352 (2000).
12. Saga, Y. et al. MesP1 is expressed in the heart precursor cells and required for the formation of a single heart tube. Development 126, 3437-3447 (1999).
13. Bondue, A. et al. Mesp1 acts as a master regulator of multipotent cardiovascular progenitor specification. Cell Stem Cell 3, 69-84 (2008).
14. David, R. et al. MesP1 drives vertebrate cardiovascular differentiation through Dkk-1-mediated blockade of Wnt-signalling. Nat Cell Biol 10, 338-345 (2008).
15. Lindsley, R.C. et al. Mesp1 coordinately regulates cardiovascular fate restriction and epithelial-mesenchymal transition in differentiating ESCs. Cell Stem Cell 3, 55-68 (2008).
16. Chan, S.S. et al. Mesp1 patterns mesoderm into cardiac, hematopoietic, or skeletal myogenic progenitors in a context-dependent manner. Cell Stem Cell 12, 587-601 (2013).
17. Burridge, P.W., Keller, G., Gold, J.D. & Wu, J.C. Production of de novo cardiomyocytes: human pluripotent stem cell differentiation and direct reprogramming. Cell Stem Cell 10, 16-28 (2012).
18. Murry, C.E. & Keller, G. Differentiation of embryonic stem cells to clinically relevant populations: lessons from embryonic development. Cell 132, 661-680 (2008).
19. Rao, J. et al. Stepwise Clearance of Repressive Roadblocks Drives Cardiac Induction in Human ESCs. Cell Stem Cell 18, 341-353 (2016).
20. Piccini, I., Arauzo-Bravo, M., Seebohm, G. & Greber, B. Functional high-resolution time-course expression analysis of human embryonic stem cells undergoing cardiac induction. Genom Data 10, 71-74 (2016).
21. Gee, H.Y. et al. KANK deficiency leads to podocyte dysfunction and nephrotic syndrome. J Clin Invest 125, 2375-2384 (2015).
22. Devalla, H.D. et al. Atrial-like cardiomyocytes from human pluripotent stem cells are a robust preclinical model for assessing atrial-selective pharmacology. EMBO Mol Med 7, 394-410 (2015).
23. Sanguinetti, M.C., Jiang, C., Curran, M.E. & Keating, M.T. A mechanistic link between an inherited and an acquired cardiac arrhythmia: HERG encodes the IKr potassium channel. Cell 81, 299-307 (1995).
24. Ran, F.A. et al. Double nicking by RNA-guided CRISPR Cas9 for enhanced genome editing specificity. Cell 154, 1380-1389 (2013).
25. Lian, X. et al. Robust cardiomyocyte differentiation from human pluripotent stem cells via temporal modulation of canonical Wnt signaling. Proc Natl Acad Sci U S A 109, E1848-1857 (2012).
26. Burridge, P.W. et al. Chemically defined generation of human cardiomyocytes. Nat Methods (2014).
27. Liu, P. et al. Requirement for Wnt3 in vertebrate axis formation. Nat Genet 22, 361-365 (1999).
28. Takada, S. et al. Wnt-3a regulates somite and tailbud formation in the mouse embryo. Genes Dev 8, 174-189 (1994).
29. Naito, A.T. et al. Developmental stage-specific biphasic roles of Wnt/beta-catenin signaling in cardiomyogenesis and hematopoiesis. Proc Natl Acad Sci U S A 103, 19812-19817 (2006).
30. Pawlowski, M. et al. Inducible and Deterministic Forward Programming of Human Pluripotent Stem Cells into Neurons, Skeletal Myocytes, and Oligodendrocytes. Stem Cell Reports 8, 803-812(2017).
31. Cowan, C.A. et al. Derivation of embryonic stem-cell lines from human blastocysts. N Engl J Med 350, 1353-1356 (2004).
32. Frank, S., Zhang, M., Scholer, H.R. & Greber, B. Small molecule-assisted, line-independent maintenance of human pluripotent stem cells in defined conditions. PLoS One 7, e41958 (2012).
33. Cong, L. et al. Multiplex genome engineering using CRISPR/Cas systems. Science 339, 819-823 (2013).
34. McLean, C.Y. et al. GREAT improves functional interpretation of cis-regulatory regions. Nat Biotechnol 28, 495-501 (2010).

## Claims

1. A method for the production of cells having a cardiomyocyte phenotype, comprising:
(a) overexpression of EOMES in embryonic stem cells or induced pluripotent stem cells for about 1 to 6 days, preferably 2 to 4 days and most preferably about 3 days, and
(b) 24h to 96h, preferably 48 to 72h after the overexpression of (a), inhibition of the WNT-pathway for at least 24h
thereby differentiating said embryonic stem cells or induced pluripotent stem cells into cells having a cardiomyocyte phenotype.

2. The method of claim 1, wherein said embryonic stem cells have been obtained from a subject or are derived from a cell line.

3. The method of claim 1 or 2, wherein the WNT-pathway is inhibited by a compound selected from the group consisting of a small molecule inhibitor, a nucleotide-based inhibitor and an amino acid-based inhibitor.

4. The method of claim 3, wherein the nucleotide-based inhibitor is an aptamer, a ribozyme, a siRNA, a shRNA or an antisense oligonucleotide and the amino acid-based inhibitor is an aptamer, an antibody or a protein drug.

5. The method of claim 3, wherein the small molecule inhibitor is selected from Wnt-C-59, XAV-939, ICG-001, IWR-1-endo, LGK-974, LF3, CP21R7 (also known as CP21), NCB-0846, PNU-74654, Salinomycin, KYA1797K, IQ-1, PRI-724, KY02111, IWP-2, IWP-L6, FH535, WIKI4, SKL2001, Wnt agonist 1IWR-1, TNKS-656 and Compd 10.

6. The method of any one of claims 1 to 5, wherein the embryonic stem cells or induced pluripotent stem cells are EOMES tet-on cells and the overexpression of EOMES is induced in the embryonic stem cells or the induced pluripotent stem cells by tetracycline or a derivative thereof.

7. The method of any one of claims 1 to 6, wherein the embryonic stem cells or the induced pluripotent stem cells are EOMES knock-out cells.

8. The method of any one of claims 1 to 7, further comprising 24h to 96h, preferably 48 to 72h after the overexpression of (a) the supplementation of retinoic acid (RA) or a synthetic agonist of the RA pathway for at least 24h, preferably for 24 to 72 hours.

9. The method of any one of claims 1 to 8, wherein the cells having a cardiomyocyte phenotype are **characterized by** one or more of the capability of synchronized beating, sarcomeric structural organization, self-contractile behavior, capability of force generation, the capability of generating action potentials, functional expression of cardiac ion channels, and the capability of electrical coupling with one another.

10. The method of any one of claims 1 to 9, wherein the cells having a cardiomyocyte phenotype are **characterized by** the expression of at least one gene being selected from NKX2.5, TNNT2, TNNC1, TNNI, ACTC1, ACTN2, MYH6, MYH7, MYL3, MYL4, MYL7, MYL2, MYOM1, RYR2, CACNA1C, CKM, KCNH2, KCNQ1, KCNA5, SCN5A, IRX4, DHRS9and NPPA.

11. The method of any one of claims 1 to 10, wherein the cells having a cardiomyocyte phenotype are **characterized by** a significantly reduced expression of at least one gene being selected from CASQ2, MYOM3 and COX7A1 as compared to naturally occurring cardiomyocytes or cultured cardiomyocytes that were obtained from naturally occurring cardiomyocytes.

12. The method of any one of claims 1 to 11, further comprising isolating a single cell having a cardiomyocyte phenotype obtained by the method of any one of claims 1 to 11 and optionally clonally expanding said cell.

13. A cell obtained by the method according to any one of claims 1 to 12.

14. The cell obtained by the method according to any one of claims 1 to 12 for use in medicine or medical or pharmaceutical research.

15. A method for identifying a compound having an pharmacological, cytotoxic, proliferative, transforming, differentiating, proarrhythmic, action potential modulating, calcium signaling modulating, electrical field potential modulating, or beating rate modulating effect on cells having a cardiomyocyte phenotype obtained by the method according to any one of claims 1 to 12, comprising:
(a) contacting said cells having a cardiomyocyte phenotype with a test compound; and
(b) determining whether the test compound has one or more of said effects on said cells having a cardiomyocyte phenotype.
